Europäisches Patentamt

European Patent Office

Office européen des brevets

(11) Publication number: **0 227 169 B1**

# EUROPEAN PATENT SPECIFICATION

(45) Date of publication of patent specification: **17.03.93**

(51) Int. Cl.5: **G01N 33/68**, G01N 33/569, C12N 15/00, C12N 5/00, A61K 39/21, C07K 15/14, C12P 21/00

(21) Application number: **86202213.4**

(22) Date of filing: **08.12.86**

(54) **Immunochemical reagent.**

(30) Priority: **17.12.85 US 809872**
**08.05.86 US 861900**
**18.07.86 US 887294**
**14.11.86 US 927577**

(43) Date of publication of application:
**01.07.87 Bulletin 87/27**

(45) Publication of the grant of the patent:
**17.03.93 Bulletin 93/11**

(84) Designated Contracting States:
**AT BE CH DE ES FR GB GR IT LI LU NL SE**

(56) References cited:
**EP-A- 0 181 150**
**EP-A- 0 226 903**
**WO-A-86/06099**
**GB-A- 2 188 639**

**SCIENCE, vol. 228, 05 April 1985; N.T.CHANG et al., pp. 93-96**

**SCIENCE, vol. 227, 01 February 1985; R.SANCHEZ-PESCADOR et al., pp. 484-492**

(73) Proprietor: **AKZO N.V.**
**Velperweg 76**
**NL-6824 BM Arnhem(NL)**

(72) Inventor: **Berman, Michael L.**
**10608 Trotters Trail**
**Potomac MD 20854(US)**
Inventor: **Crush, Sylvia A.**
**13000 Country Ridge**
**Germantown MD 20874(US)**
Inventor: **Wong-Staal, Flossie**
**3 Lynn Manor CT**
**Rockville MD 20850(US)**
Inventor: **Gallo, Robert C.**
**8513 Thorndon Terrace**
**Bethesda MD(US)**

(74) Representative: **Hermans, Franciscus G.M. et al**
**Patent Department AKZO N.V. Pharma Division P.O. Box 20**
**NL-5340 BH Oss (NL)**

EP 0 227 169 B1

**Description**

The present invention is concerned with an immunochemical reagent and with polypeptide mimicking amino acid sequences of AIDS-related viral proteins.

The disease AIDS (acquired immune deficiency syndrome) only recently has been recognized as a rapidly spreading affliction with often dramatic and devastating consequences for the victims. It has also been recognized that AIDS is spread by viral infections and this causative virus has been named HTLV-III (Science, 224, 497-508, 1984). Alternative names like LAV and ARV-2 were also coined by various investigators to name their isolates of the virus causing AIDS. In the present application the name HTLV-III will be used throughout to encompass also LAV, ARV-2 and all other variants causing AIDS. The thus named variants were shown to be very closely related with regard to their genomic organization.

Nevertheless, by cloning and molecular characterization of HTLV-III, it was shown also that independent isolates of the virus have substantial diversity of DNA sequence, as reflected in the differences of their restriction enzyme cleavage patterns. These observations, combined with DNA sequence analysis, have led to the general belief that point mutants responsible for genetic diversity and genetic drift in HTLV-III may contribute to the pathogenicity of this virus. Such genetic drift has been shown to account for increased pathogenicity of Visna virus isolates (Clements et al., PNAS, 77: 4454-4458, 1980). Likewise, recurrent febrile episodes of encephalitis caused by equine encephalitis virus may be the result of mutations arising in the infected population (Montelaro et al., Journal of Biological Chemistry, 259: 10539-10544, 1980). The virus HTLV-III, although shown to be distinct from other RNA viruses by nucleic acid sequence determination, is clearly a member of the retrovirus family.

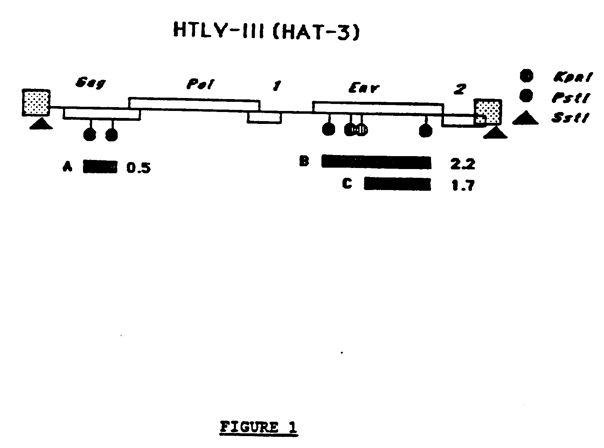

The general genomic organisation of HTLV-III viral nucleic acid is shown in Figure 1. This figure shows three principal genes of the virus. Gag is the group capsid antigens precursor gene; pol is the reverse transcriptase gene; and env is the gene of the precursor to the envelope glycoproteins. Two additional open reading frames (ORF's), labelled 1 and 2, are indicated. The gene of the transactivating factor of HTLV-III corresponds to ORF1. The function of any gene product from ORF2 is unknown. The boxed areas at the ends of Figure 1 represent the viral long terminal repeats (LTRs).

The products of the three principal viral genes can be characterized briefly as follows:

I. gag Gene Products

Positioned at the 5' end of the genome, the gag open reading frame sequence encodes a 55K $M_r$ - (molecular mass) precursor protein. This precursor is processed into three mature proteins. Amino acids 1 through 132 correspond to a 17K virion protein and is probably involved in morphogenesis. The next mature protein is the major capsid structural protein, p24. The remainder of the gag poly-protein (p15), a highly basic protein, is probably involved in nucleic acid binding.

II. pol Gene Products

The predicted amino acid sequence of the second long open reading frame from HTLV-III shows homology and similarity with other pol genes of other retroviruses. This 3,044 nucleotides long open reading frame has the potential to code for a 1,015 amino acid precursor protein for the reverse transcriptase present in HTLV-III virion. In addition to the 5' reverse transcriptase information, there exists 3' genetic information probably involved in endonuclease and integrase functions necessary for viral replication.

III. env Gene Products

The env-lor region of HTLV-III encodes a precursor protein about 863 amino acids long. This primary gene product, $M_r$ 160K, can be detected in infected cells. This precursor is cleaved into two products, a hydrophilic exterior protein (gp120) that is glycosylated and a hydrophobic transmembrane segment. The virion gp41 corresponds to the transmembrane protein. There is a high degree of conservation of amino acid sequence at the transmembrane protein processing site. The regions outside of this are less well conserved and show considerable difference at the nucleotide and amino acid sequence level when different viral isolates are compared.

Once various isolates of HTLV-III had been cloned by different investigators, segments of the genome, as defined by DNA sequence analysis, were targeted for genetic engineering. Published work includes genetic engineering and expression in E.coli of varying segments of the env-gene of HTLV-III. Viral isolate BH-10 was expressed in open reading frame vectors (Chang et al., Science, 228: 93-95, 1985; Chang et al., Biotechnology, 3: 905-909, 1985). Similarly, the env-gene of viral isolate HXB-3 was cloned and expressed in E.coli by Crowl et al. (Cell, 41: 979-986, 1985).

The expression in E.coli of a portion of the LAV genome encoding gag proteins and the use of these proteins in vaccines, is described in WO 86/06099. EP 181150 describes the expression of hTLR DNA sequences, e.g. the sequences encoding gag p25 and gag p16, in E.coli and the use of the recombinant proteins for diagnostic purposes.

WO86/06099 and EP 181150, however, do not mention recombinant gp41 and gp160 env gene products.

In order to halt the presently observed vigorous spreading of AIDS, routine testing of blood for the presence of the virus or antibodies against it, was introduced on a massive scale. The tests currently in use for detection of antibodies against HTLV-III are based on one or more immunoreagents containing antigenic material derived from the native virus.

An at least equally important instrument in halting the spreading of the disease will be a vaccine effectively protecting the groups at risk in contacting the virus against infection with HTLV-III.

Both for immuno-diagnostic and for vaccination purposes there is a recognized need for effective antigenic material derived from a safe source, hence not derived directly from the native virus.

The method of choice for the preparation of this (polypeptide) antigenic material is hence the synthetic route, in particular by organic chemical synthesis, or by recombinant DNA technology.

It is the purpose of the present invention to provide an immunochemical reagent which can be put into use in the above-mentioned applications.

This immunochemical reagent is characterized in that it comprises a combination of two or more synthetic polypeptide sequences selected from the group consisting of
- a polypeptide sequence mimicking at least one antigenic determinant of the gag-antigen of HTLV-III;
- a polypeptide sequence mimicking at least one antigenic determinant of the glycoprotein gp120 of HTLV-III; and
- a polypeptide sequence mimicking at least one antigenic determinant of the glycoprotein gp41 of HTLV-III.

With a combination of polypeptide sequences is meant here any physical or chemical composition of said polypeptides, such as a single synthetic polypeptide containing said two or more polypeptide sequences, or a mixture of synthetic polypeptides each containing at least one of said polypeptide sequences.

As indicated above the term synthetic here means derived from any source other than the natural one. Hence, it implies among others: prepared by organic chemical synthesis (in particular, for the present polypeptides this implies one of the well known methods of polypeptide synthesis, such as the solid phase method according to Merrifield), or prepared by expression of a recombinant polynucleotide containing a polynucleotide sequence coding for one or more of the present polypeptides in a host microorganism.

The polypeptide sequences mimicking said antigenic determinants may be present as such in the immuochemical reagent according to the invention, they may be bonded to each other, and/or to additional polypeptide sequences, and/or to a suitable carrier, etc. This is largely dependent upon the intended use of the immunochemical reagent (vaccination or immuno-diagnosis).

A vaccine according to the invention comprises the immunochemical reagent and generally also a so-called adjuvant, to promote the immune response of the vaccinated individual. Suitable adjuvants are for example Freunds' incomplete and complete, Corynebacterium parvum, Bordetella pertussis, muramyl dipeptide, saponins, alhydrogel or mineral oils.

The immunochemical reagent in the vaccine optionally may comprise one kind of carrier, to which each of the polypeptide sequences are bonded or a number of carriers, each differing in the nature of the polypeptide sequences bonded. These carriers may for example be liposomes, or micelles of e.g. saponins or the like, or proteins such as key hole limpet hemocyanin or (bovine) serum albumin, or other viruses or microorganisms on whose surface the polypeptide sequences according to the invention are exposed (the latter also often named vector-vaccines).

For use in an immunochemical assay method the immunochemical reagent may either be applied free in solution; or may comprise a solid entity or a labelling entity. Said solid entity may, for example, be a microtitration plate, or a tube, a stick, a rod, a filter, a bead or a solid particle, such as a latex particle, an erythrocyte or a particle of a sol of a dye, a metal or a metal compound. As a labelling entity use can be made, for example, of a radio-active atom or compound, a fluorescent compound, a phosphorescent compound, an enzyme, or a small solid particle such as a particle of a solf of a dye, a metal, or a metal compound. For the purpose of the present invention an antigenic determinant is considered the minimum structural element of an antigen that enables interaction with antibodies that also recognize the native antigen. For the antigenic determinant as an isolated entry this implies that it should permanently or transiently present the same spatial arrangement which the same element possesses in the native antigen.

Such a polypeptide antigenic determinant comprises generally at least about 4 or 5 amino acids. In a linear antigenic determinant these at least 4 or 5 amino acids are arranged as a relatively short polypeptide chain, whereas in a conformational antigenic determinant the at least 4 or 5 amino acids forming the determinant generally are distributed scattered over a considerable length of a polypeptide chain that folds and bends in such a way that the relevant amino acids are placed in each other's proximity. Both these types of antigenic determinants form part of the present invention.

The polypeptide sequences mimicking the antigenic determinants of the gag-antigen, the glycoprotein gp120 and the glycoprotein gp41, respectively should be substantially free of the naturally occurring gag, gp120 and gp41 proteins. Even though, the immunochemical reagent according to the invention optionally also may contain one or more of the naturally occurring HTLV-III proteins.

Preferably, one or more of the polypeptides may comprise only a relevant portion of the respective proteins. In particular, the antigenic determinants of the glycoproteins gp120 and gp41 may according to the present invention suitably be mimicked by a portion of these proteins comprising at least about 4 to about 160 amino acids.

These portions can be selected by any of the methods known in the art for the selection of antigenic determinants polypeptide sequences, or by employing the method to be described in the examples to follow.

After having selected the relevant portions of polypeptides advantageously by the method to be described below, these selected polypeptides can be synthetically prepared, as already explained by organic chemical coupling of the amino acids or by employing well-known recombinant DNA technology. In either of the two cases the at least two polypeptides according to the invention may be prepared as isolated polypeptides or as fused polypeptides. These fused polypeptides either may comprise at least two of the two or more synthetic polypeptides in one sequence, or may comprise a non-related polypeptide sequence, or a combination of both. A suitable non-related polypeptide sequence may, for example, be an enzyme, which in an enzyme-immuno-assay may serve as the label.

In the investigation underlying the present invention use was made of a method for the systematic study of viral gene antigenicity by expressing parts of those genes as fusion proteins in transformed cells. The various fusion proteins were tested using various methods (Western blot, Elisa) to assess their reactivity with respect to various antisera (anti-p24, anti-p41 and anti-p120). Although the following examples used transformed E.coli to illustrate the invention, any viable transformed cell line that effectively expresses the subject fusion protein could be used according to techniques known to those of ordinary skill in the art. Although our method has some similarities to those disclosed in the previously cited HTLV-III cloning studies, none of these publications allows a systematic picture of antigenic determinants to be assembled. The previously published data using the recombinant antigens made in E.coli demonstrate immunoreactivity with sera from patients with AIDS. However, unlike the study underlying the present invention, no conclusions could be drawn about regions of the env-gene not represented in the cited clones.

The starting point for this study was a new HTLV-III isolate named HAT-3. The DNA of this virus was cloned into a phage-derived vector as outlined in the specific examples to follow.

In Figure 1 restriction sites for this λHAT-3 prophage are indicated. Bars A, B and C refer to segments of the gag and env genes cloned in the examples. The numbers refer to the sizes of these DNA fragment in kilobases. Sequence information for these portions of λHAT-3 is given in Figures 2 and 3.

In Figure 2 the DNA sequence and the polypeptide sequence derived therefrom of fragment A (see Figure 1) from gag are presented.

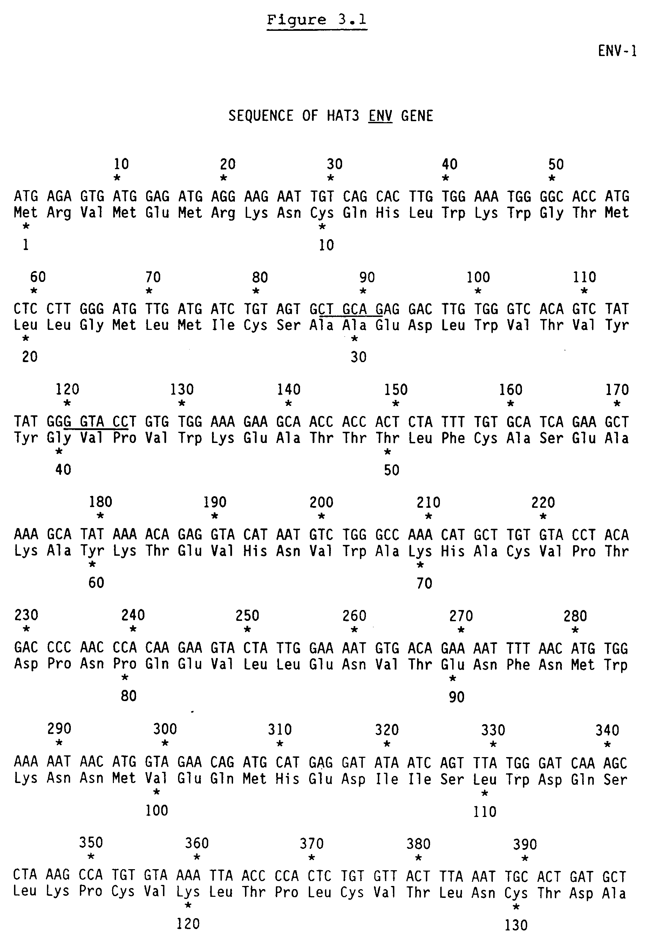

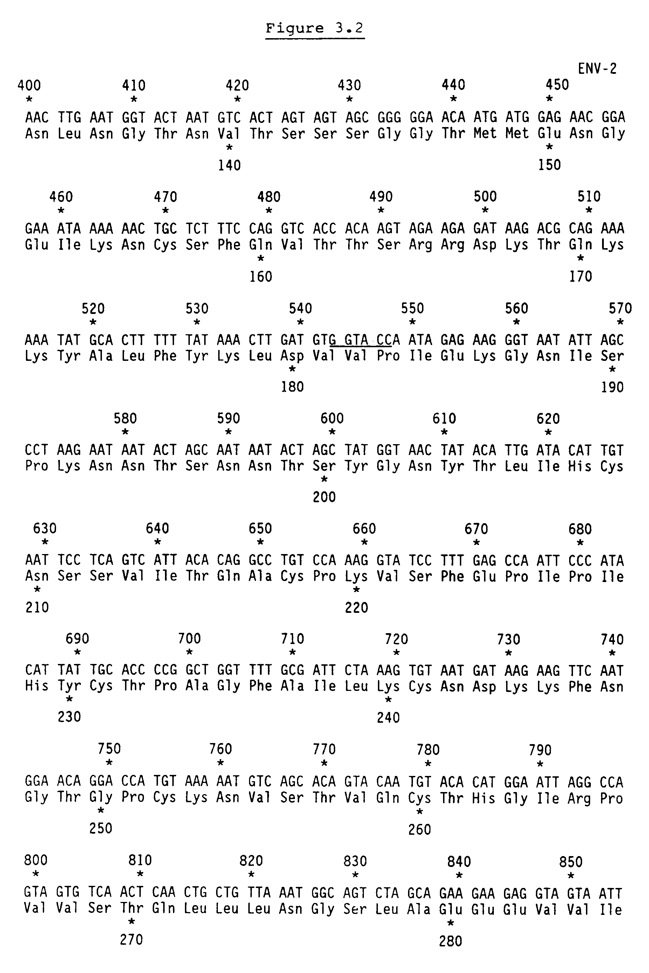

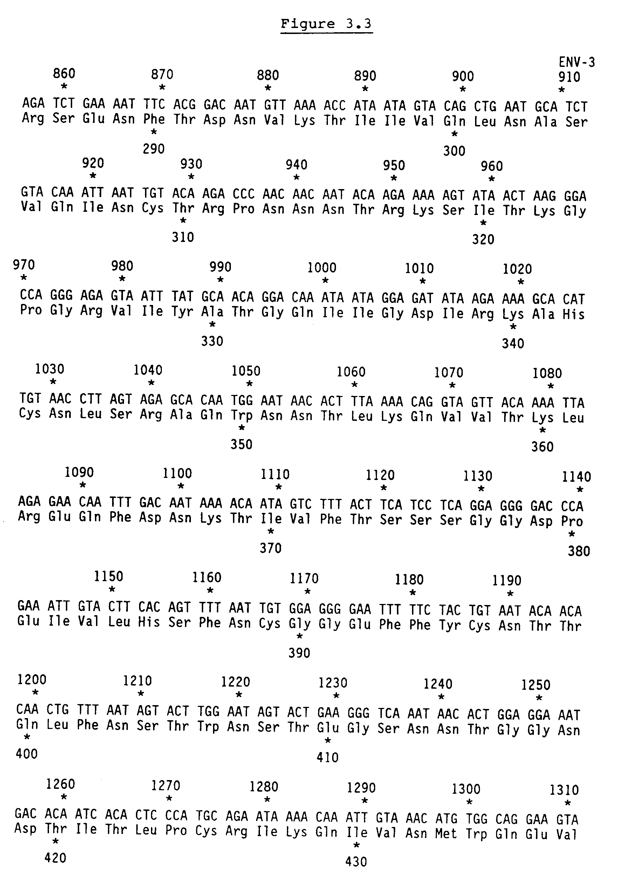

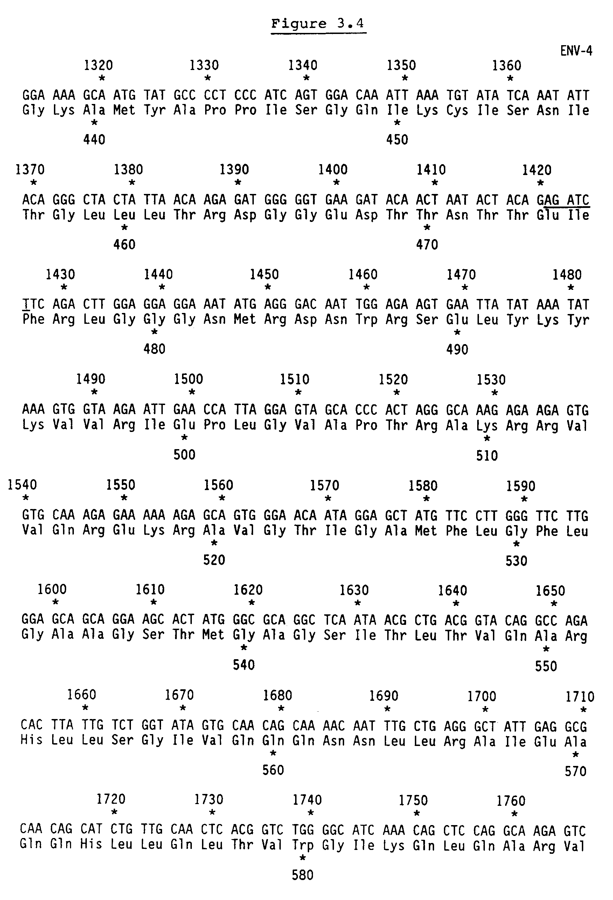

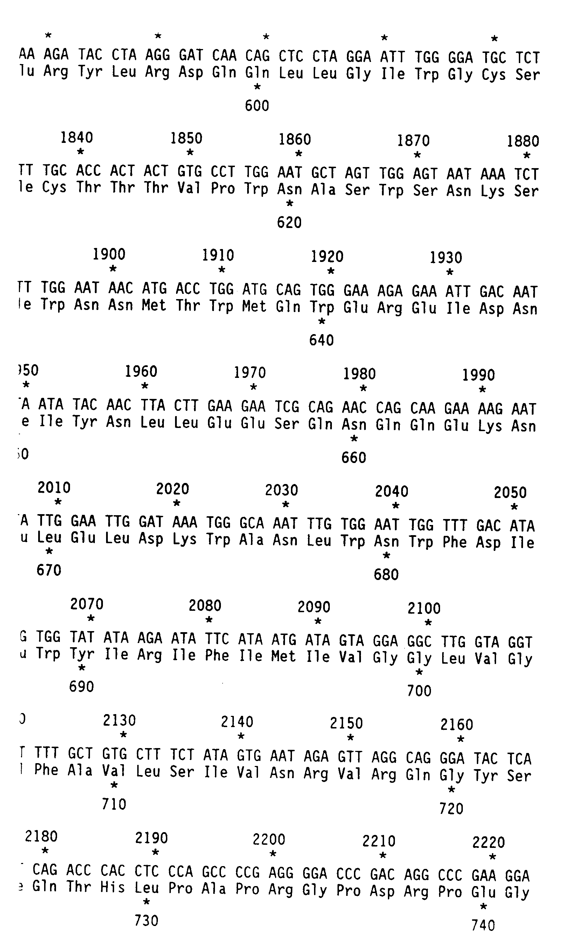

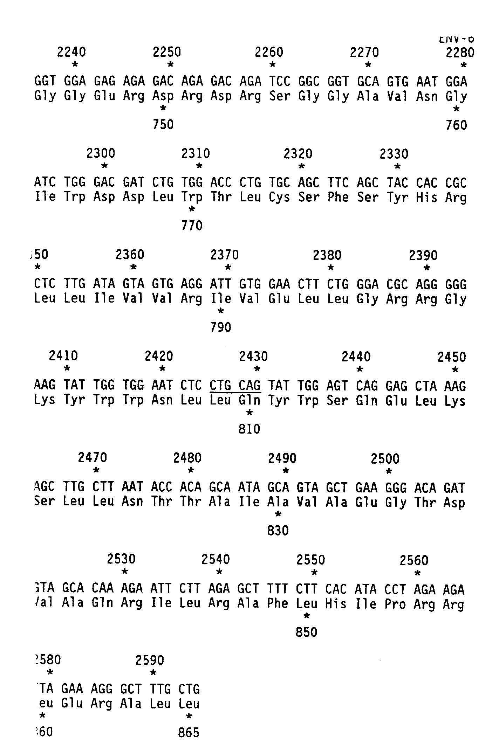

The DNA sequence of the entire p24 structural gene is given. The extent of DNA in fragment A is indicated by the regions between the two PstI (CTGCAG) restriction enzyme sites. Figure 3 reports the DNA sequence and the derived polypeptide sequence of fragments B and C (see Figure 1) from env. The DNA sequence of the entire env gene is given. Fragment B spans the DNA between the two indicated PstI sites. Fragment C starts at the (second) indicated KpnI site and terminates at the same PstI site as does fragment B. Underlined sites include PstI = CTGCAG, KpnI = GGTACC, and BglII = AGATCT.

Although similar in size and length to other env-genes that have been sequenced, we undertook our systematic study using this gene.

As a result of the study described herein we were able to identify several immuno-dominant sections of the protein coded by the env-gene. It was found that immunochemical reagents according to the invention advantageously could comprise peptides corresponding with or mimicking said immuno-dominant sections.

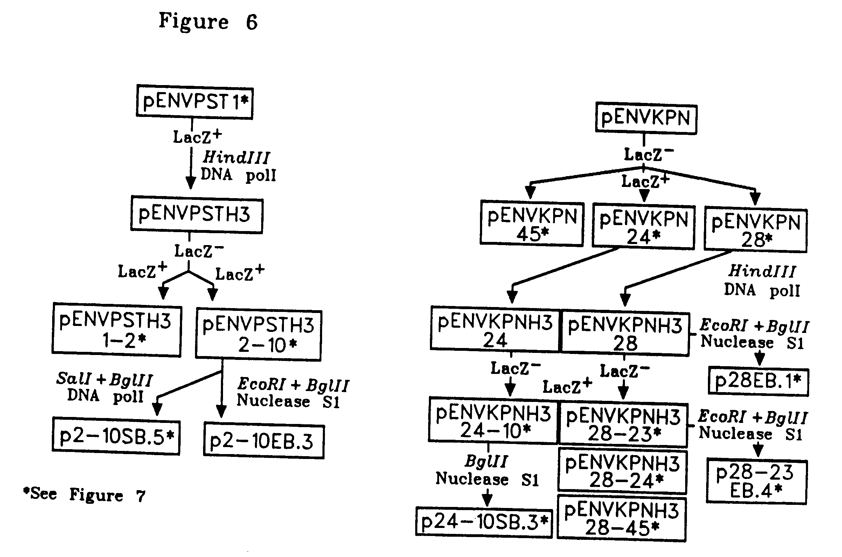

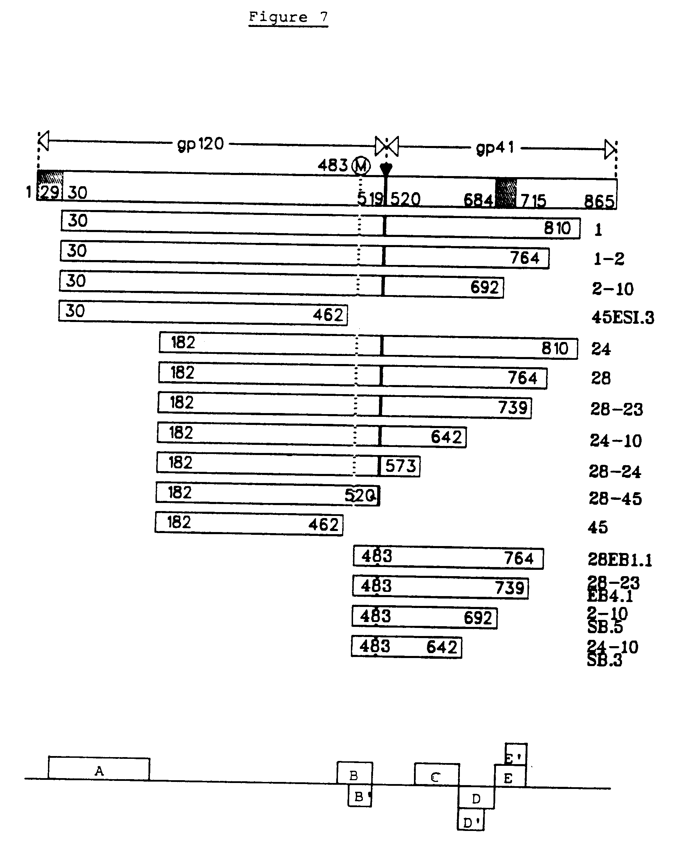

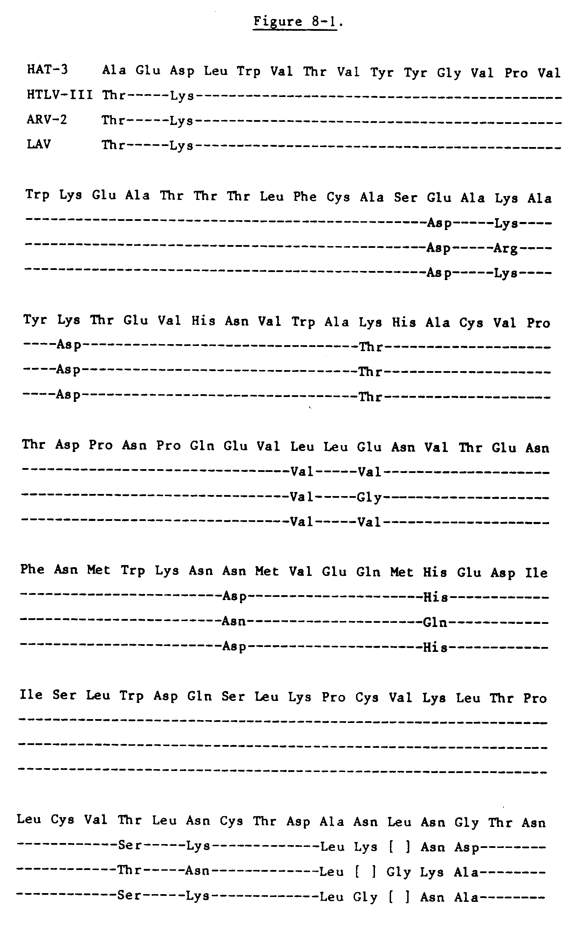

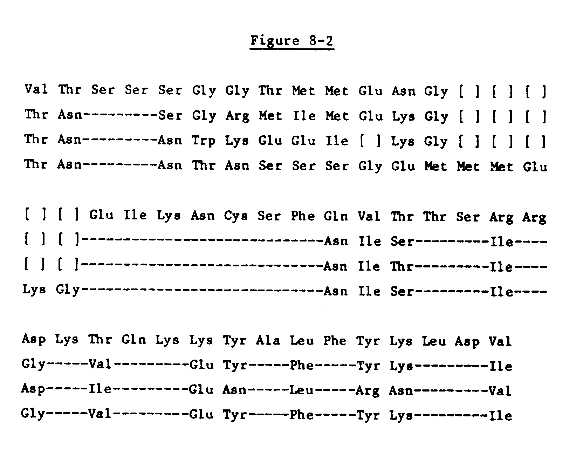

A suitable immuno-dominant section was identified to be comprised within the amino acid sequence extending between amino acids No. 30 and 181 inclusive of the env open reading frame of HAT-3 (bar A at

EP 0 227 169 B1

the bottom of Figure 7), and that corresponds for example with amino acids No. 38 to 188 inclusive of HTLV-III (Ratner et al., Nature 313, 277-284; 1985); with amino acids No. 38-187 inclusive of ARV-2 (Sanchez-Pescador et al., Science 227, 484-492; 1985), and with amino acids No. 38-193 inclusive of LAV (Wain-Hobson et al., Cell 40, 9-17; 1985). These amino acid sequences are depicted in Figure 8.

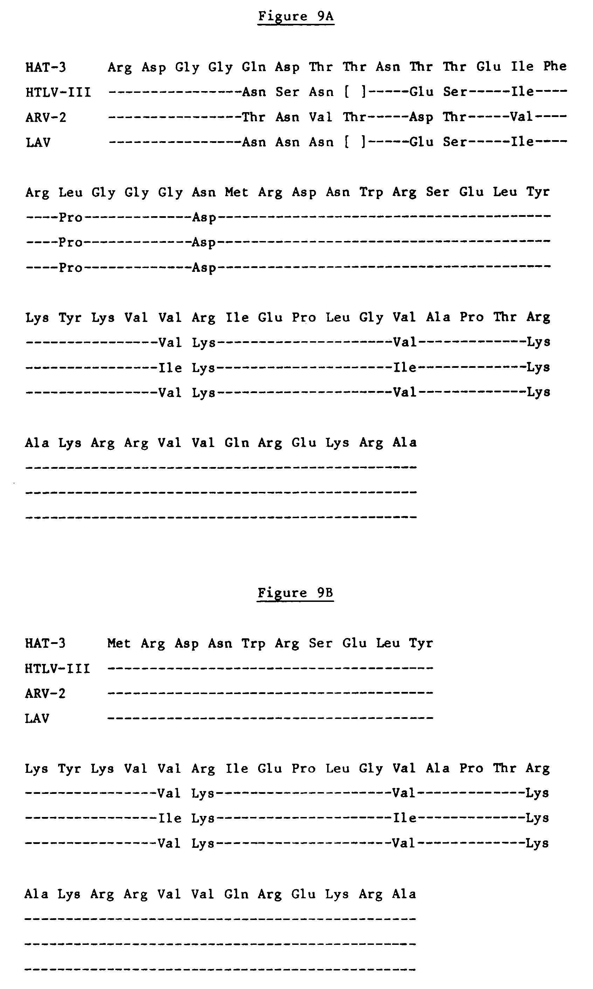

Another suitable immuno-dominant section was identified to be comprised within the amino acid sequence extending between amino acids No. 463 and 520 inclusive and more particular between amino acids No. 483 and 520 inclusive of HAT-3 (bar B and B[1] respectively at the bottom of Figure 7), that corresponds for example with amino acids No. 463-519 (more particularly No. 482-519) of HTLV-III, with amino acids No. 461-518 (more particularly No. 481-518) of ARV-2, and with amino acids No. 468-524 (more particularly No. 487-524 of LAV) (Figures 9A and 9B, respectively).

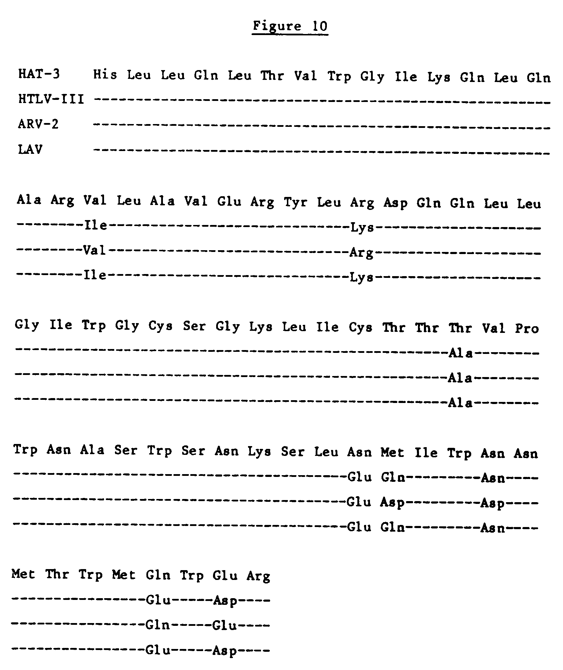

A further immuno-dominant section was identified to be comprised within the amino acid sequence extending between amino acids No. 473-642 of the env open reading frame of HAT-3 (bar C at the bottom of Figure 7), that corresponds for example with amino acids No. 571-640 of the env open reading frame of HTLV-III, with the amino acids No. 571-640 of the env open reading frame of ARV-2, and with the amino acids No. 576-645 of the env open reading frame of LAV. These four corresponding sequences are represented in Figure 10.

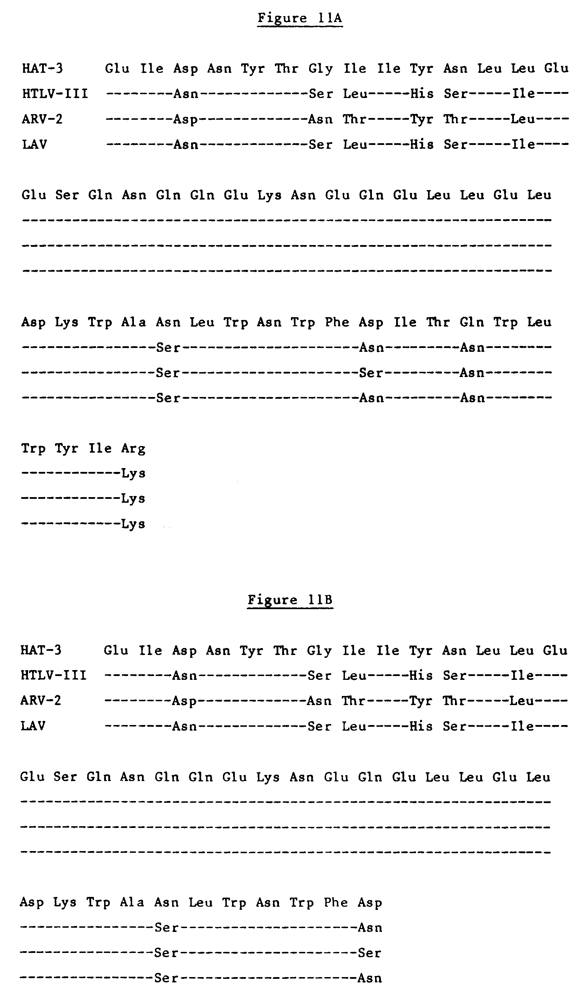

A still further immuno-dominant section was identified to be comprised within the amino acid sequence extending between amino acids No. 643-692 and more particularly between amino acids No. 643-683 of the env open reading frame of HAT-3 (bar D and D[1], respectively, at the bottom of Figure 7), that corresponds for example with amino acids No. 641-690 (more in particular No. 641-681) of the env open reading frame of HTLV-III, with the amino acids No. 641-690 (more in particular No. 641-681) of the env open reading frame of ARV-2, and with the amino acids No. 646-695 (more in particular No. 646-686) of the env open reading frame of LAV. These four corresponding sequences are represented in Figures 11A and 11B, respectively.

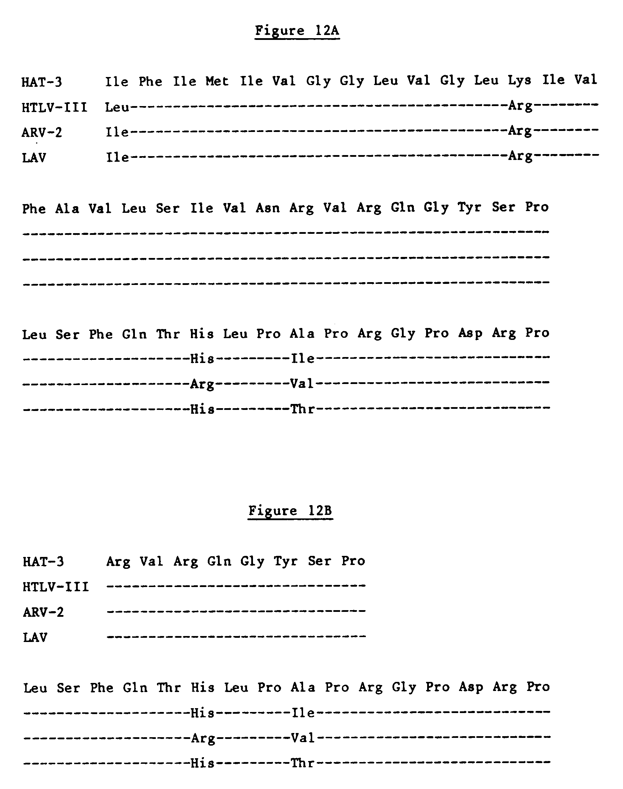

Still another immuno-dominant section was identified to be comprised within the amino acid sequence extending between amino acids No. 693-739 and more particularly between amino acids No. 716-739 of the env open reading frame of HAT-3 (bar E and E[1], respectively, at the bottom of Figure 7), that corresponds for example with amino acids No. 691-737 (more in particular No. 714-737) of the env open reading frame of HTLV-III, with the amino acids No. 691-737 (more in particular No. 714-737) of the env open reading frame of ARV-2, and with the amino acids No. 696-742 (more in particular No. 719-742) of the env open reading frame of LAV. These four corresponding sequences are represented in Figures 12A and 12B, respectively.

Plasmids p24-10SB.3, pENVKPN45 and pGAG8, and HTLV-III isolate HAT-3 virus were deposited in the American Type Culture Collection, 12301 Parklawn Drive, Rockville, Maryland 20852 on December 17th, 1985, prior to filing this patent application, and assigned ATCC Deposit Nos. 53375, 53374, 53373 and 40213, respectively.

Plasmids p2-10SB.5, p28-23EB.4, p28-EB.1 and p45ESI.3 were deposited in the American Type Culture Collection on July 18, 1986, prior to filing this patent application, and assigned ATCC Deposit Nos. 67159, 67157, 67158 and 67160, respectively.

Example 1

Host strain

DH5 Δ(lac)U169. This E.coli strain is a derivative of DH1 (ATCC, Rockville, Maryland), which is lac[+] - (Hanahan, D., J. Mol. Biol. (1983) 166: 557).
Standard E.coli genetic techniques were used to introduce the Δ(lac)U169 deletion. This deletion completely removes the genes of the lac operon and any homology with the plasmids. The parent strain DH5 is a spontaneous isolate of DH1 that has a 10-fold higher transformation frequency compared to DH1. The complete genotype of this strain is: DH5, F[−] Δ(argF - lac)U169 endA1 hsdR17($r_k^−$, $m_k^+$) supE44 thi-1 λ - reCA1 gyrA96 relA1.

Example 2

Cloning vector

The cloning vectors used in these experiments are derivatives of pBR322 (ATCC, Rockville, Maryland). All HTLV-III clones are derived from plasmid pMLB1113.IGl. This plasmid carries a portion of E.coli lac

5

operon cloned between the EcoRI site (nucleotide 4361) and the AvaI site (nucleotide 1425) of pBR322. The lac are oriented in a clockwise direction in the standard pBR322 representation. The lacZ gene fragment originated from plasmid pMLB1034 (Berman et al., U.S. Patent 4,503,142). The lacI gene fragment originated from plasmid pMC9 (Calos, Nature, 274, 762-765; 1978). These genes are cloned from a naturally occurring HincII site preceding the lacI gene to a naturally occurring AvaI site at codon 70 in lacY. This corresponds to nucleotides (n.t.) 13-4667 of the pMLB1113.IGI sequence. (The annotated sequence of the lac operon has been assembled in Appendix M, pages 273-281, of Silhavy et al., supra.)

In this vector, several changes have been introduced into the lac genes via standard genetic engineering techniques. There is a point mutation in the promoter for lacI (W.T. C → T; n.t. 28 of pMLB1113.IG1) that results in 10X higher levels of repressor synthesis (LacI$^{q1}$). At codon 5 in the lacZ gene, the multiple cloning sites from plasmid PUC8 (Vieira and Messing, Gene, 19: 259-268, 1982) have been incorporated (n.t. 1310-1345 of pMLB1113.IG1). The naturally occurring EcoRI site at codon 1006 of lacZ is removed by a point mutation (W.T. C → A; n.t. 4352 of pMLB1113.IG1.

In addition to alterations to the lac genes, a single strand 1 phage origin has been incorporated into the pBR322 sequence. A 514 base pair fragment from bacteriophage M13 (ATCC, Rockville, Maryland) has been cloned between two adjacent AhaIII sites distal to the bla gene (PBR322 n.t. 3232 to n.t. 3251 replaced with M13 n.t. 5488 to n.t. 6001; n.t. 6472 to n.t. 6985 of pMLB1113.IGI). This M13 region functions to generate single-stranded DNA from the pBR322 derivative in the presence of a helper M13 phase (Zagursky and Berman, Gene (1984) 27: 183-191). These functions have no effect on normal pBR322 replication or maintenance and are only active during M13 phage infection.

The parenteral cloning vector, pMLB1113.IGI, confers ampicillin resistance and highly regulated synthesis of native $\beta$-galactosidase to the host cell DH5 $\Delta$(lac). A Lac$^-$ derivative of this vector was used for several of the actual cloning experiments. This derivative was altered at the BamHI site (n.t. 1320) by cleavage and fill-in with DNA polymerase. This plasmid is designated pMLB1120.IGI. All the alterations to these cloning vectors have been verified by DNA sequence analysis of the recombinant regions.

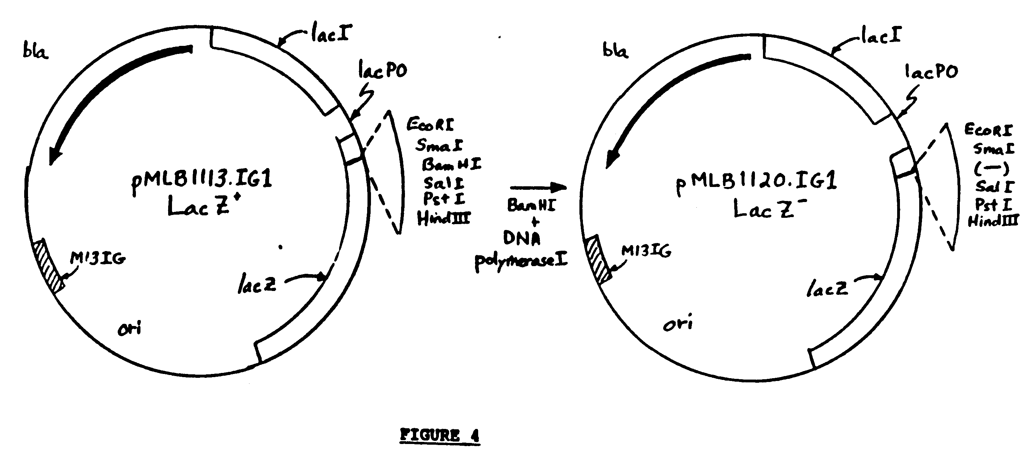

The two cloning vectors (pMLB1113.IGI and pMLB1120.IGI) used in this study are diagrammed in Figure 4. Solid boxes represent the lac genes of E.coli. The M13 intergenic region is indicated by the striped box. The gene encoding ampicillin resistance (bla) and the origin of replication (ori) from pBR322 is indicated. A frameshift mutation ( + 1) was introduced at the multiple cloning site as indicated. This mutation was confirmed by determining the DNA sequence at the cloning site.

## Example 3

### HTLV-III DNA

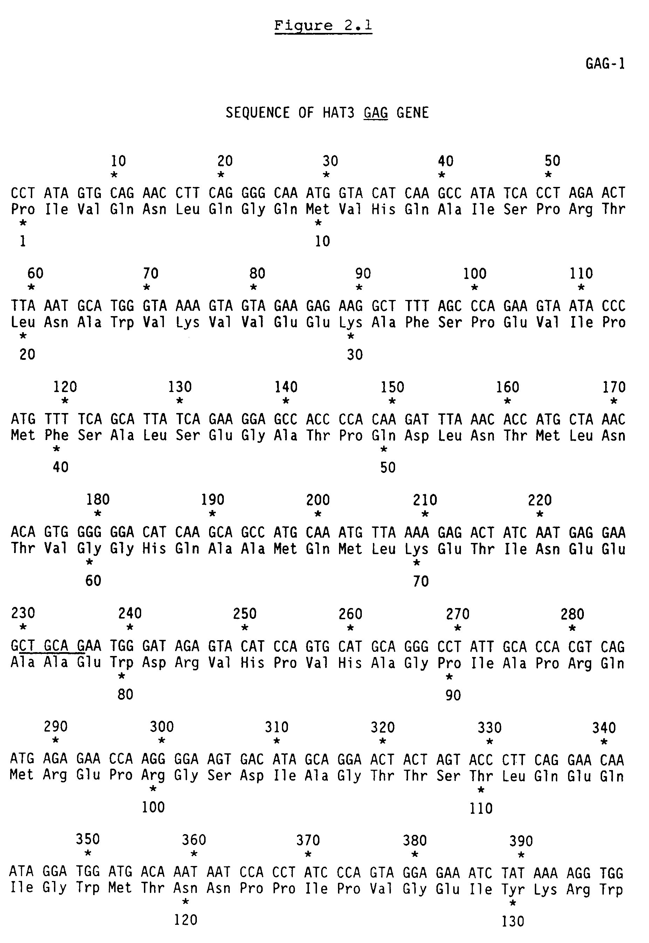

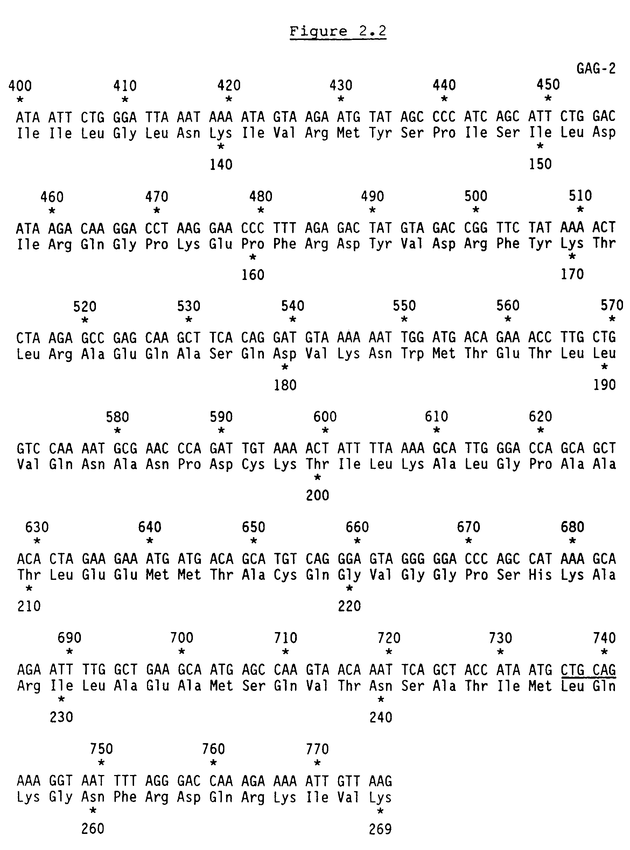

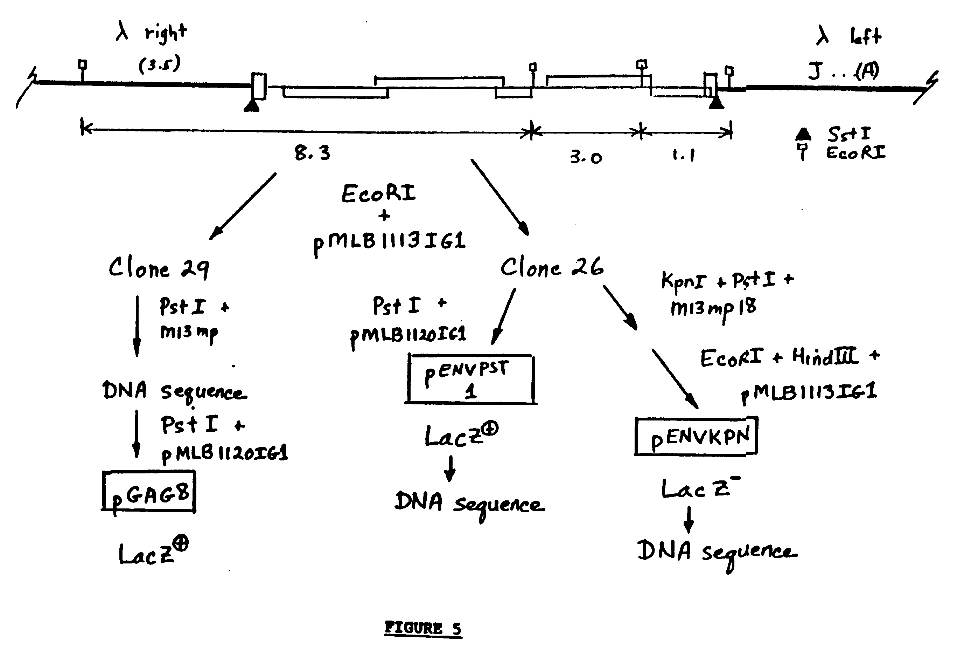

A genomic clone (λHAT-3) of an HTLV-III prophage in bacteriophage was used. This particular clone carries an approximately 9Kb HTLV-III DNA fragment from two naturally occurring SstI sites in the viral LTRs. A simplified restriction enzyme site map of this clone is shown in Figure 1. Unpublished DNA sequence information from the gag and env gene regions is included here (Figures 2 and 3). This allowed us to obtain certain restriction enzyme fragments from the original clone and various subclones constructed in our lab (Figure 5). A partial map is shown on the top line of Figure 5. The sizes of the EcoRI fragments in this region of the clone are indicated. These fragments were subcloned into plasmid vector pMLB1113.IG1. Clone 29 carries the 8.3 Kbp EcoRI fragment from the phage. Clone 26 carries the 3.0 Kbp and 1.1 Kbp EcoRI fragments. Digestion of these subclones with the appropriate enzymes (as indicated) yielded the three starting plasmids enclosed in boxes.

## Example 4

### Plasmid pGAG8 (see Figure 5)

This clone is derived from plasmid pMLB1120.IG1 by the insertion of a 506 base pair Pst1 fragment from the p24 coding region of HTLV-III (see Figure 2). This DNA encodes a 169 amino acid polypeptide internal to the p24 portion of gag, which represents approximately 70% of the p24 gene product.

Following induction of the operon with $10^{-3}$M isopropylthiogalactoside (IPTG), the 134K MW hybrid protein was produced in an amount of approximately 10-15% of total cellular protein. An equal amount of native size $\beta$-galactosidase monomer was also synthesized. In cell sonicates centrifuged at a low speed (~10,000 x g), the hybrid Gag-LacZ protein could be isolated in insoluble form in the pellet.

This protein was resolubilized in 7M Guanidine-HCl and maintained in solution of 0.05M Guanidine-HCl.

6

## Example 5

### Plasmid pENVPST1

This clone is derived from plasmid pMLB1120.IG1 by the insertion of a 2339 base pair PstI fragment from the env gene of HTLV-III (see Figure 3). This fragment represents 94% of the viral coding region. (The coding sequences for gp41 are also carried on this fragment, see above.)

This clone is the poorest producer of HTLV-III hybrid proteins. Following induction, it produces about 1% Env-LacZ hybrids. The full size fusion protein (206K MW) is the least abundant, but reacts strongly with positive patient's serum. A major hybrid protein appears at the 149K MW immuno-reactive band. Two additional $\beta$-galactosidase hybrids, synthesized in variable amounts, appear at 133K and 124K MW positions. Finally, a substantial band is present at the position of $\beta$-galactosidase. All four size classes of hybrid proteins reacts with anti-$\beta$-galactosidase antiserum, while the two highest molecular weight species react with HTLV-III positive patient serum.

## Example 6

### Plasmid pENVKPN24 and Related Plasmids

This clone is derived from an internal KpnI - PstI fragment cloned into phage M13mp18 Gene 33, 103-109; 1985and subsequently subcloned (using EcoRI - HindIII) into pMLB1113.IGl. The resulting clone (pENVKPN) is LacZ⁻ because the translational frame of env and lacZ are not the same. Various spontaneous mutants, called "pop-ups", were isolated using a published protocol (Berman and Jackson, J. Bacteriol., 159: 750-756, 1984). One of these, pENVKPN24, synthesized about 5% levels of the Env-LacZ hybrid. This clone differed from the pENVPST1 clone in that 152 codons have been removed from between the 5′ PstI and KpnI sites (deletion of 457 n.t.). DNA sequence analysis of this clone revealed a deletion of an A residue located at n.t. 3225 of pENVKPN24 that re-aligns the env coding region with the lacZ coding segment. Unlike the pENVPSTI clone, pENVKPN24 produces mostly full size hybrids (~200K MW) and a second, less abundant protein at ~149K MW. This plasmid was converted to LacZ⁻ by enzymatically introducing a frameshift mutation at the HindIII site adjacent to lacZ. Once again, various LacZ⁺ derivatives were selected. (See Figure 6).

A second Lac⁺ derivative of plasmid pENVKPN selected the same way is plasmid pENVKPN45. This plasmid synthesized about 10-15% levels of Env-LacZ hybrid. In this clone, DNA sequence analysis revealed a deletion of 1024 n.t.

A third Lac⁺ derivative of plasmid pENVKPN from this selection is plasmid pENVKPN28. This plasmid synthesized high levels of a 200K M.W. hybrid at levels similar to pENVKPN24. The same method used for pENVKPN24, was used to engineer a LacZ⁻ derivative of pENVKPN28. Various Lac⁺ mutants had suffered deletions of env genetic material. DNA sequence analysis of these derivatives showed that all Lac⁺ mutants had suffered deletions of env genetic material. The extent of these deletions is summarized in Figures 6 and 7.

A fourth Lac⁺ derivative p45ESI.3 was constructed by exchanging the EcoRI-StuI 587 bp fragment from clone pENVPST1 for the 118 bp EcoRI-StuI fragment of pENVKPN45.

## Example 7

### High level Production of Recombinant Antigens Corresponding to (g)p120, (g)p41 and p24 Viral Gene Products

Several in vitro deletions were introduced enzymatically into the LacZ⁺ clones in order to obtain high levels (1-15% of total cellular protein) of recombinant hybrids. The level of protein production from plasmid pGAG8 was sufficient as constructed. This plasmid produces the equivalent of recombinant p24 from our particular viral isolate.

Plasmid pENVKPN45 produces the equivalent of (g)p120 as a $\beta$-galactosidase hybrid at a 10-15% level. Plasmids pENVKPNH3 28-45 and p45ESI.3 are additional sources of the recombinant g(p120)hybrid proteins. The pENVKPNH3 28-45 and p45ESI.3 clones express $\beta$-galactosidase hybrid proteins at 5-10% of total cellular protein.

Plasmid pENVPSTH3 2-10 and pENVKPNH3 24-10 were subjected to restriction nuclease digestion and religation. (See Figure 6.)

The top of Figure 7 represents a map of the env gene of HTLV-III. The three striped areas of hydrophobic regions corresponding to the signal sequence (far left), a region at the N-terminus of the p41 precursor, and a transmembrane anchor segment (far right). Vertical lines at codon positions 30, 144 and 810 correspond to the cloning sites PstI, KpnI and PstI, represented as nucleotide positions 112, 544, 2454, respectively in Figure 3. The site of proteolytic cleavage that yields p120 (left-most) and p41 is indicated by the ∇ symbol.

The right-most column indicates the number of the clone (refer to Figure 6). The open bars indicate the extent of DNA from env in the individual clones. The numbers inside of these bars refer to the first codon in env and the last codon in env (using the pENVPST1 sequence as a reference). These details were determined using DNA sequence information.

The four bars p28EB1.1, p28-23EB4.1, p2-10SB.5 and p24-10SB.3 represent "DNA" encoding four additional gene products from pENVPST1 (no. 1). Internal initiation of protein synthesis begins at the naturally occurring methionine codon 483, represented by the dotted vertical lines. This is represented by (M) on the Figure. All other bars represent precise alterations to the DNA sequence resulting in the production of hybrid proteins of the indicated molecular weight. All hybrid proteins show a positive reaction with anti-$\beta$-galactosidase anti-serum. Cysteine residues are represented by c's above the env map. The vertical dashed lines indicate the location of two BglII sites used for genetic engineering. (See Figure 6).

Example 8

Immunochemical testing of HAT-3 env-derived polypeptides

The env-derived polypeptides produced according to Example 7 were tested with regard to their immunochemical properties both using ELISA and Western blot. For these tests use was made of solubilized material from the harvested cells.

A. Solubilization of env-derived recombinant polypeptides

Harvested cells are suspended in a buffer containing 50 mmol/l of Tris, 0.1 mmol/l of PMSF, pH 7.8, in a ratio of 1 gram of cells: 4 ml of the buffer. Cells are lysed using a French pressure cell at an internal working pressure of 20,000 PSI. The lysed cell suspension is evacuated from the pressure cell at a flow rate of 15 drops/minute into a chilled vessel and centrifuged at 48,000 xg for 30 minutes at 4 °C. The resulting pellet material (called ($P_1$) is washed with a solution containing 50 mmol/l of Tris and 0.1 mmol/l of PMSF and centrifuged as described above. The passed $P_1$ pellet material is then homogeneously suspended, using a spatula or rubber policeman, into a solution containing 8 mol/l urea, 50 mmol/l of Tris, 10 mmol/l of EDTA, 1 mmol/l of DH, 0.1 mmol/l of PMSF with a pH of 8.1; the ratio of grams of initial cells used for $P_1$: ml of solubilizing buffer is 1:4. This suspension is sonicated using a sonicating water bath at room temperature for 45 minutes. The solution is centrifuged at 200,000 xg for 45 minutes at 4 °C. The resulting supernatant, called $S_2$, may be dialyzed against PBS or against a solution containing 50 mmol/l of Tris, 10 mmol/l of EDTA, 1 mmol/l of DH and 0.1 mmol/l of PMSF, of pH 8.1 to removed urea, or may be left in the urea buffer. The thus prepared fraction $S_2$ was used for the ELISA and Western blot analysis.

B. ELISA

The ELISA is performed according to the following steps:

1. The wells of a microtitration plate are coated with 100 $\mu$l of antigen obtained by dialysing the $S_2$ fraction obtained according to A. against 0.05 mmol/l sodium carbonate/bicarbonate buffer, pH 9.6. The coating is performed at 4 °C with 0.5-10 $\mu$g/ml, during 1-3 days, depending upon the antigen preparation.

2. The antigen is aspirated, and the wells are blocked with Blotto (5% non fat dry milk in $dH_2O$). This is coated for 45 minutes at room temperature.

3. While the plates are being blocked the anti-sera containing antibodies specific against HTLV-III antigens which have been diluted 1:100 with Blotto + 10% goat serum/PBS + 1% Triton® X-100 are incubated for 30 minutes at 37 °C with a $S_2$ preparation of the E.coli which does not contain a vector coding for HTLV-III antigens.

4. Blotto is aspirated from the wells, 100 $\mu$l of the absorbed diluted antiserum is added and the microtitration plate is incubated for 90 minutes in a heating block at 37 °C.

5. The serum is aspirated and the wells are washed thrice with 1% glycerol + 0.05% Tween® 20.

6. Subsequently 100 μl of a conjugate solution containing 0.143 μg goat-anti-human IgG(H + L)/HRP (Kirkegaard and Perry Laboratories, Gaithersburg, MD) per ml of 10% goat serum/PBS + 10% Triton® X-100 are added to each well, and incubated for 60 minutes in a heating block at 37 °C.

7. The conjugate is aspirated and the wells are washed six times.

8. 100 μl of TMB is added to each of the wells of the microtitration plate and allowed to react during 30 minutes at room temperature. Thereafter the reaction is stopped by the addition of 100 μl of a 4N $H_2 SO_4$ solution and the absorbance is read at 450 nm.

## C. Western blot analysis

For this analysis the HTLV-III antigens of $S_2$ are first separated by SDS-PAGE using a method according to Laemmli (Nature 227, 680-5; 1970), and the thus separated antigens are subsequently transferred to nitrocellulose sheets, according to the method described by Towbin et al. (Proc.Natl.Acad.Sci. 76, 4350-4).

## D. Reactivity of Hybrid Proteins

Hybrid proteins expressed by plasmids listed in the first column of Table I were coated to microtitration plates and showed the reactivity indicated in Table I with Serum 1 and Serum 2 containing antibodies to gp41 and both gp41 and gp120 proteins, respectively, in the test system described under B. These results confirm the reactivity of peptide sequences indicated by the bars A, B, B[1] and C in Figure 7. The higher molecular weight hybrid proteins illustrate protein synthesis beginning with the lac operon (e.g., 206 M.W. hybrid) and the lower molecular weight hybrids are proteins for which synthesis is initiated with the naturally occurring methionine codon 483.

### TABLE I

| Plasmid | M.W. hybrid | Serum 1 (gp41+) | Serum 2 (gp41+ and gp120+) |
|---|---|---|---|
| pENVPST 1 | 206-149K | + | + |
| pENVPSTH3 1-2 | 200-149K | + | + |
| pENVPSTH3 2-10 | 200-140K | + | + |
| p45ES1.3 | 185K | − | + |
| pENVKPN 24 | 200-150K | + | + |
| pENVKPN 28 | 200-149K | + | + |
| pENVKPNH3 28-23 | 190-145K | + | + |
| pENVKPNH3 24-10 | 180-133K | + | + |
| pENVKPNH3 28-24 | 155-118K | − | + |
| pENVKPNH3 28-45 | 152-117K | − | + |
| pENVKPN 45 | 150K | − | − |

## E. The use of a combination of recombinant proteins in a screening assay for antibodies against HTLV-III

a. A number of sera from individuals at risk of contracting an infection with HTLV-III was tested in an ELISA, using the procedure described under B. Plates were coated with HTLV-III recombinant proteins

(S$_2$ fractions prepared as described under A.). Plates a. were coated with proteins (2.5 $\mu$g/ml) from clone 24-10SB.3, plates b. were coated with proteins (1.5 $\mu$g/ml) from clone gag-8 and plates c. were coated with a mixture of the two proteins (2.5 $\mu$g/ml 24-10SB.3 plus 1.5 $\mu$g/ml gag-8).

The results were compared with data obtained by the Western blot (WB) technique described under C. and a commercial ELISA (Vironostika), both using natural viral lysate as the source of antigen (Table II).

## TABLE II

| Plates: | a | | b | | c | | | VIRONOS- | |
|---|---|---|---|---|---|---|---|---|---|
| antigen: | 24-10SB3 | | gag-8 | | combination | | WB | TIKA | |
| sample | | | | | | | | | |
| 1 | .24 | + | .27 | – | .61 | + | 41 | 1.04 | + |
| 2 | .19 | – | 1.26 | + | 1.32 | + | 24,41 | 0.30 | + |
| 3 | .46 | + | .31 | – | .88 | + | 41 | 2.65 | + |
| 4 | 1.79 | + | .41 | + | 2.51 | + | 41,24 | >3.00 | + |
| 5 | 1.41 | + | .74 | + | 2.50 | + | 41,24 | >3.00 | + |
| 6 | .33 | + | .53 | + | .80 | + | 24 | .12 | – |
| 7 | .22 | – | .36 | – | .55 | – | neg. | .20 | – |
| Low positive | | | | | | | | | |
| control | .39 | + | .62 | + | 1.52 | + | 41,24 | 1.31 | + |
| NHS | .14 | – | .29 | – | .40 | – | neg. | .12 | – |
| Cut off | | | | | | | | | |
| value | .24 | – | .39 | – | .60 | – | – | .68 | |

The above data show that a test system wherein a combination of two antigenic polypeptides according to the invention are employed performs more reliably than a test system with a single type of antigenic polypeptide.

b. Plates d. were coated with a combination of S$_2$ fractions of three recombinant proteins (clones gag-8 (1.5 $\mu$g/ml), 24-10SB.3 (2.5 $\mu$g/ml) and p45 ESL3 (2.5 $\mu$g/ml) by methods described above. A number of sera, previously analyzed for anti-HTLV-III antibodies by Western blot and ELISA (Vironostika) techniques, using natural viral lysate as the source of antigen, was treated on these plates, as well as on the plates with gag-8 and 24-10SB.3 antigen (plate c. described.

## Table III

| Plates: | d | c | VIRONOSTIKA |
|---|---|---|---|
| coated with: | 3 antigens | 2 antigens | viral lysate |
| Samples (n) | | | |
| Western blot positive 100 | 99 pos. | 89 pos. | 95 pos. |

It thus appears that a test system wherein the combination of three antigenic polypeptides employed scores significantly more of the samples that were found to be positive by Western blot analysis.

F. The effect of the length of the polypeptide chain of the p41 recombinant protein

Plates were coated with $S_2$ preparations of the following clones: plates e. with 2-10 SB.5 (2.5 μg/ml), plates f. with 28 EB1.1 (2.5 μg/ml) and plates g. with 28.23 EB4.1 (2.5 μg/ml). Sera were tested using these plates by the methods described above. The results (Table IV) are compared with those obtained on plates a. (coated with clone 24-10 SB3) and with Western blot.

### Table IV

| Plate: | a | | e | | f | | g | | Western blot on viral lysate |
|---|---|---|---|---|---|---|---|---|---|
| antigen: | 24-10 SB.3 | | 2-10 SB.5 | | 28 EB1.1 | | 28-23 EB4.1 | | |
| Samples | | | | | | | | | |
| 11 | 0.23 | – | 2.30 | + | 2.83 | + | >3.00 | + | 41,24 |
| 12 | 0.7 | – | .27 | – | .34 | – | .48 | – | 41 weak |
| 13 | .19 | – | 1.05 | + | .78 | + | .94 | + | 41,24 |
| 14 | .15 | – | .85 | – | 1.47 | + | 1.95 | + | 41,24 |
| 15 | .24 | + | 1.20 | + | 1.44 | + | 1.93 | + | 41 |
| 16 | .46 | + | >3 | + | >3 | + | >3 | + | 41 |
| 17 | .24 | + | 1.42 | + | 1.68 | + | 2.00 | + | 41 |
| NHS | .14 | | .82 | | .68 | | .63 | | |
| Cut off value | .24 | | .92 | | .78 | | .73 | | |

These data indicate that some of the sera which are unreactive with plate a. show positive reaction with plates e.,f. and g. From this result it is concluded that the extra polypeptide fragment in clone 2-10 SB.5 (bar D in Figure 7) is responsible for the binding of an extra class of antibodies, and hence that this polypeptide fragment comprises another antigenic determinant.

Another difference is detected with sample 14, which was negative both with plate a. and e., and significantly positive with both plates f. and g. This result suggests the presence of another antigenic determinant comprised within the extra polypeptide fragment in clone 28 EB1.1 as compared to clone 2-10 (bar E in Figure 7).

Furthermore it is tempting to assume that the transmembrane segment of gp41 (the shaded area in the uppermost bar of Figure 7 between amino acids No. 684 and 715) will not be involved in the binding of antibodies in vivo. From this assumption it is inferred that the antigenic determinants of D and E are comprised in the segments indicated by bars D[1] and E[1], respectively.

**Claims**

1. Immunochemical reagent characterized in that it comprises a combination of two or more synthetic polypeptide sequences selected from:
   - a polypeptide sequence containing at least one antigenic determinant of the gag-antigen of HTLV-III isolate λHAT-3;
   - a polypeptide sequence containing at least one antigenic determinant of the glycoprotein gp120 of HTLV-III isolate λHAT-3; and

- a polypeptide sequence containing at least one antigenic determinant of the glycoprotein gp41 of HTLV-III isolate λHAT-3, said isolate comprising genomic DNA from HTLV-III isolate HAT-3 virus deposited with the ATCC under No. 40213.

2. Immunochemical reagent according to claim 1, characterized in that it comprises a polypeptide corresponding to the carboxyterminal about 58 amino acids long polypeptide of the gp120 protein.

3. Immunochemical reagent according to claim 2, characterized in that it comprises a polypeptide having comparable immunochemical properties as a polypeptide of the sequences represented in figure 9A.

4. Immunochemical reagent according to claim 1, characterized in that it comprises a polypeptide corresponding to the carboxyterminal about 38 amino acids long polypeptide of the gp120 protein.

5. Immunochemical reagent according to claim 4, characterized in that it comprises a polypeptide having comparable immunochemical properties as any of the polypeptides of the sequences represented in figure 9B.

6. Immunochemical reagent according to any one of claims 1 to 5, characterized in that it comprises a polypeptide having comparable immunochemical properties as the partial amino acid sequence extending between amino acid no. 53 and amino acid no. 122 of the gp41 protein.

7. Immunochemical reagent according to claim 6, characterized in that it comprises a polypeptide having comparable immunochemical properties as a polypeptide of the sequences represented in figure 10, corresponding to amino acids 571-640 of the HTLV-III sequence.

8. Immunochemical reagent according to any one of claims 1 to 7, characterized in that it comprises a polypeptide having comparable immunochemical properties as the partial amino acid sequence extending between amino acid no. 123 and amino acid no. 172 of the gp41 protein.

9. Immunochemical reagent according to claim 8, characterized in that it comprises a polypeptide having comparable immunochemical properties as a polypeptide of the sequences represented in figure 11A, corresponding to amino acids 641-690 of the HTLV-III sequence.

10. Immunochemical reagent according to any one of claims 1 to 7, characterized in that it comprises a polypeptide having comparable immunochemical properties as the partial amino acid sequence extending between amino acid no. 123 and amino acid no. 163 of the gp41 protein.

11. Immunochemical reagent according to claim 10, characterized in that it comprises a polypeptide having comparable immunochemical properties as a polypeptide of the sequences represented in figure 11B, corresponding to amino acids 641-681 of the HTLV-III sequence.

12. Immunochemical reagent according to any one of claims 1 to 11, characterized in that it comprises a polypeptide having comparable immunochemical properties as the partial amino acid sequences extending between amino acid no. 173 and amino acid no. 217 of the gp41 protein.

13. Immunochemical reagent according to claim 12, characterized in that it comprises a polypeptide having comparable immunochemical properties as a polypeptide of the sequences represented in figure 12A, corresponding to amino acids 691-737 of the HTLV-III sequence.

14. Immunochemical reagent according to any one of claims 1 to 11, characterized in that it comprises a polypeptide having comparable immunochemical properties as the partial amino acid sequence extending between amino acid no. 196 and amino acid no. 217 of the gp41 protein.

15. Immunochemical reagent according to claim 14, characterized in that it comprises a polypeptide having comparable immunochemical properties as a polypeptide of the sequences represented in figure 12B, corresponding to amino acids 714-737 of the HTLV-III sequence.

16. Immunochemical reagent according to any one of claims 1 to 15, characterized in that it comprises a polypeptide having comparable immunochemical properties as the partial amino acid sequence extending between amino acid no. 30 and amino acid no. 181 of the gp120 protein.

17. Immunochemical reagent according to claim 16, characterized in that it comprises a polypeptide having comparable immunochemical properties as a polypeptide of the sequences represented in figure 8.

18. Synthetic polypeptide comprising at least one amino acid sequence having the immunochemical properties of the amino acid sequence of HTLV-III isolate λHAT-3, said isolate comprising genomic DNA from HTLV-III isolate HAT-3 virus deposited with the ATCC under No. 40213 which polypeptide is selected from:
    - the carboxyterminal 38 amino acids long polypeptide of the gp120 protein;
    - the partial amino acid sequence extending between amino acid no. 53 and amino acid no. 122 of the gp41 protein;
    - the partial amino acid sequence extending between amino acid no. 123 and amino acid no. 163 of the gp41 protein;
    - the partial amino acid sequence extending between amino acid no. 196 and amino acid no. 217 of the gp41 protein; and
    - the partial amino acid sequence extending between amino acid no. 30 and amino acid 181 of the gp120 protein.

19. Synthetic polypeptide according to claim 18 comprising at least one of the amino acid sequences represented in the figures 8, 9A, 9B, 10, 11A, 11B, 12A and 12B, corresponding to a part of the λHAT-3 sequence.

20. A recombinant DNA characterized in that it comprises a DNA sequence coding for the production of a polypeptide according to claim 18 or 19.

21. A plasmid characterized in that it comprises a recombinant DNA according to claim 20.

22. A host cell characterized in that it is transformed by the plasmid according to claim 21.

23. The HTLV-III isolate λHAT-3, comprising genomic DNA from HTLV-III isolate HAT-3 virus deposited with the ATCC under No. 40213.

24. A plasmid according to claim 21, characterized in that it is derived from the HTLV-III isolate of claim 23.

25. The plasmid of claim 24, selected from the group consisting of p24-10SB.3, pENVKPN45, pGAG8, p2-10SB.5, p28-23EB.4, and p28-EB.1.

26. The plasmid of claim 24, selected from the group consisting of pENVPST1, pENVPSTH3, pENVPSTH3 1-2, pENVPSTN3 2-10, p2-10 EB.3, pENVKPN, pENVKPN24, pENVKPNH3 24, pENVKPNH3 24-10, pENVKPN 28, pENVKPNH3 28, pENVKPNH3 28-23, pENVKPNH3 28-24, and pENVKPNH3 28-45.

27. A method for preparing the plasmid of claim 21, comprising:
    (a) inserting a cDNA sequence from HTLV-III into a plasmid for transforming a cellular host;
    (b) deleting from the plasmid of step (a) a portion of an identifiable, expressable sequence to effect an out-of-phase frameshift for that sequence;
    (c) transforming a cellular host with the plasmid of step (b);
    (d) culturing the transformed host;
    (e) selecting the host cells showing spontaneous mutations that effect an in-phase frameshift for the identifiable sequence of step (b);
    (f) culturing the host cell selected in step (e);
    (g) selecting the host cell that produce useful amounts of polypeptides that are effectively immunoreactive with HTLV-III antibodies; and
    (h) isolating and identifying plasmids that code for the polypeptides of step (g).

**28.** A polypeptide characterized in that it is expressed by host cell transformed with a plasmid according to claim 25 or 26.

**29.** A test kit for use in an immunoassay characterized in that it comprises an immunochemical reagent according to any one of claims 1 to 17.

**Patentansprüche**

**1.** Immunochemisches Reagens, dadurch gekennzeichnet, dass es eine Kombination von zwei oder mehr synthetischen Polypeptidsequenzen enthält, ausgewählt aus :
   - einer Polypeptidsequenz, welche mindestens einen antigenen Determinanten des gag-Antigens von HTLV-III-Isolat λHAT-3 enthält;
   - eine Polypeptidsequenz, welche mindestens einen antigenen Determinanten des Glycoproteins gp120 von HTLV-III-Isolat λHAT-3 enthält, und
   - eine Polypeptidsequenz, welche mindestens einen antigenen Determinanten des Glycoproteins gp41 von HTLV-III-Isolat λHAT-3 enthält, welches Isolat genomische DNA aus HTLV-III-Isolat HAT-3-Virus, hinterlegt bei der ATCC unter der No. 40213, umfasst.

**2.** Immunochemisches Reagens nach Anspruch 1, dadurch gekennzeichnet, dass es ein Polypeptid entsprechend dem carboxyendständigen, etwa 58 Aminosäuren langen Polypeptid des gp120-Proteins enthält.

**3.** Immunochemisches Reagens nach Anspruch 2, dadurch gekennzeichnet, dass es ein Polypeptid enthält, welches vergleichbare immunochemische Eigenschaften wie ein Polypeptid der in Figur 9A dargestellten Sequenzen aufweist.

**4.** Immunochemisches Reagens nach Anspruch 1, dadurch gekennzeichnet, dass es ein Polypeptid entsprechend den carboxyendständigen, etwa 38 Aminosäuren langen Polypeptid des gp120-Proteins enthält.

**5.** Immunochemisches Reagens nach Anspruch 4, dadurch gekennzeichnet, dass es ein Polypeptid enthält, welches vergleichbare immunochemische Eigenschaften wie die Polypeptide der in Figur 9B dargestellten Sequenzen aufweist.

**6.** Immunochemisches Reagens nach einem der Ansprüche 1 bis 5, dadurch gekennzeichnet, dass es ein Polypeptid enthält, welches vergleichbare immunochemische Eigenschaften wie die partielle Aminosäuresequenz aufweist, welche sich zwischen Aminosäure No. 53 und Aminosäure No. 122 des gp41-Proteins erstreckt.

**7.** Immunochemisches Reagens nach Anspruch 6, dadurch gekennzeichnet, dass es ein Polypeptid enthält, welches vergleichbare immunochemische Eigenschaften wie ein Polypeptid der in Figur 10 dargestellten Sequenzen, entsprechend den Aminosäuren 571-640 der HTLV-III-Sequenz, aufweist.

**8.** Immunochemisches Reagens nach einem der Ansprüche 1 bis 7, dadurch gekennzeichnet, dass es ein Polypeptid enthält, welches vergleichbare immunochemische Eigenschaften wie die partielle Aminosäuresequenz aufweist, welches sich zwischen Aminosäure No. 123 und Aminosäure No. 172 des gp41-Proteins erstreckt.

**9.** Immunochemisches Reagens nach Anspruch 8, dadurch gekennzeichnet, dass es ein Polypeptid enthält, welches vergleichbare immunochemische Eigenschaften wie ein Polypeptid der in Figur 11A dargestellten Sequenzen, entsprechend den Aminosäuren 641-690 der HTLV-III-Sequenz, aufweist.

**10.** Immunochemisches Reagens nach einem der Ansprüche 1 bis 7, dadurch gekennzeichnet, dass es ein Polypeptid enthält, welches vergleichbare immunochemische Eigenschaften wie die partielle Aminosäuresequenz aufweist, welche sich zwischen Aminosäure No. 123 und Aminosäure No. 162 des gp41-Proteins erstreckt.

**11.** Immunochemisches Reagens nach Anspruch 10, dadurch gekennzeichnet, dass es ein Polypeptid enthält, welches vergleichbare immunochemische Eigenschaften wie ein Polypeptid der in Figur 11B dargestellten Sequenzen, entsprechend den Aminosäuren 641-681 der HTLV-III-Sequenz, aufweist.

**12.** Immunochemisches Reagens nach einem der Ansprüche 1 bis 11, dadurch gekennzeichnet, dass es ein Polypeptid enthält, welches vergleichbare immunochemische Eigenschaften wie die partiellen Aminosäuresequenzen aufweist, welche sich zwischen Aminosäure No. 173 und Aminosäure No. 217 des gp41-Proteins erstrecken.

**13.** Immunochemisches Reagens nach Anspruch 12, dadurch gekennzeichnet, dass es ein Polypeptid enthält, welches vergleichbare immunochemische Eigenschaften wie ein Polypeptid der in Figur 12A dargestellten Sequenzen, entsprechend den Aminosäuren 691-737 der HTLV-III-Sequenz, aufweist.

**14.** Immunochemisches Reagens nach einem der Ansprüche 1 bis 11, dadurch gekennzeichnet, dass es ein Polypeptid enthält, welches vergleichbare immunochemische Eigenschaften wie die partielle Aminosäuresequenz aufweist, welche sich zwischen Aminosäure No. 196 und Aminosäure No. 217 des gp41-Proteins erstreckt.

**15.** Immunochemisches Reagens nach Anspruch 14, dadurch gekennzeichnet, dass es ein Polypeptid enthält, welches vergleichbare immunochemische Eigenschaften wie ein Polypeptid der in Figur 12B dargestellten Sequenzen, entsprechend den Aminosäuren 714-737 der HTLV-III-Sequenz, aufweist.

**16.** Immunochemisches Reagens nach einem der Ansprüche 1 bis 15, dadurch gekennzeichnet, dass es ein Polypeptid enthält, welches vergleichbare immunochemische Eigenschaften wie die partielle Aminosäuresequenz aufweist, welche sich zwischen Aminosäure No. 30 und Aminosäure No. 181 des gp120-Proteins erstreckt.

**17.** Immunochemisches Reagens nach Anspruch 16, dadurch gekennzeichnet, dass es ein Polypeptid enthält, welches vergleichbare immunochemische Eigenschaften wie ein Polypeptid der in Figur 8 dargestellten Sequenzen aufweist.

**18.** Synthetisches Polypeptid, welches mindestens eine Aminosäuresequenz enthält, welche die immunochemischen Eigenschaften der Aminosäuresequenz von HTLV-III-Isolat- λHAT-3 aufweist, welches Isolat die genomische DNA auf HTLV-III-Isolat-HAT-3-Virus, hinterlegt bei der ATCC unter der No. 40213 umfasst, wobei das Polypeptid ausgewählt ist aus:
- dem carboxyendständigen, 38 Aminosäuren langen Polypeptid des gp120-Proteins;
- der partiellen Aminosäuresequenz, welche sich zwischen Aminosäure No. 53 und Aminosäure No. 122 des gp41-Proteins erstreckt;
- der partiellen Aminosäuresequenz, welche sich zwischen Aminosäure No. 123 und Aminosäure No. 163 des gp41-Proteins erstreckt;
- der partiellen Aminosäuresequenz, welche sich zwischen Aminosäure No. 196 und Aminosäure No. 217 gp41-Proteins erstreckt; und
- der partiellen Aminosäuresequenz, welche sich zwischen Aminosäure No. 30 und Aminosäure No. 181 des gp120-Proteins erstreckt.

**19.** Synthetisches Polypeptid nach Anspruch 18, welches mindestens eine der in den Figuren 8, 9A, 9B, 10, 11A, 11B, 12A und 12B dargestellten Aminosäuresequenzen, entsprechend einem Teil der λHAT-3-Sequenz, enthält.

**20.** Eine Rekombinante DNA, dadurch gekennzeichnet, dass sie eine DNA-Sequenz enthält, welche für die Erzeugung eines Polypeptides gemäss Anspruch 18 oder 19 kodiert.

**21.** Ein Plasmid, dadurch gekennzeichnet, dass es eine Rekombinante DNA nach Anspruch 20 enthält.

**22.** Wirtzelle, dadurch gekennzeichnet, dass sie durch das Plasmid nach Anspruch 21 transformiert wird.

**23.** Das HTLV-III-Isolat-λHAT-3, enthaltend genomische DNA aus HTLV-III-Isolat-HAT-3-Virus, hinterlegt bei der ATCC unter No. 40213.

**24.** Ein Plasmid nach Anspruch 21, dadurch gekennzeichnet, dass es von HTLV-III-Isolat nach Anspruch 23 abgeleitet ist.

**25.** Das Plasmid nach Anspruch 24, ausgewählt aus der Gruppe bestehend aus p24-10SB.3, pENVKPN45, pGAG8, p2-10SB.5, p28-23EB.4 und p28-EB.1.

**26.** Das Plasmid nach Anspruch 24, ausgewählt aus der Gruppe bestehend aus pENVPST1, pENVPSTH3, pENVPSTH3 1-2, pENVPSTH3 2-10, p2-10 EB.3, pENVKPN, pENVKPN24, pENVKPNH3 24, pENVKPNH3 24-10, pENVKPN 28, pENVKPNH3 28-23, pENVKPNH3 28-24 und pENVKPNH3 28-45.

**27.** Ein Verfahren zur Herstellung des Plasmides nach Anspruch 21, umfassend :
(a) die Einführung einer cDNA-Sequenz aus HTLV-III in ein Plasmid zur Transformierung eines aus Zellen bestehenden Wirtes;
(b) Entfernung eines Teils einer identifizierbaren, exprimierbaren Sequenz aus dem Plasmid von Stufe (a), um eine ausser-Phasen-Rahmenverschiebung für die Sequenz zu bewirken:
(c) Transformieren eines aus Zellen bestehenden Wirtes mit dem Plasmid aus Stufe (b);
(d) Züchtung des transrormierten Wirtes;
(e) Auswahl der Wirtzellen, welche spontane Mutationen aufweisen, welche eine in-Phasen-Rahmenverschiebung für die identifizierbare Sequenz der Stufe (b) bewirken,
(f) Züchtung der in Stufe (e) ausgewählten Wirtzelle;
(g) Auswahl der Wirtzelle, welche nützliche Mengen an Polypeptiden erzeugt, welche mit HTLV-III-Antikörpern wirksam immunologisch reaktionsfähig sind; und
(h) Isolierung und Identifizierung von Plasmiden, welche für die Polypeptide der Stufe (g) kodieren.

**28.** Ein Polypeptid, dadurch gekennzeichnet, dass es durch Wirtzellen exprimiert wird, welche mit einem Plasmid nach Anspruch 25 oder 26 transformiert sind.

**29.** Eine Testgarnitur zur Verwendung in einem immunologischen Test, dadurch gekennzeichnet, dass sie ein immunochemisches Reagens nach einem der Ansprüche 1 bis 17 enthält.

**Revendications**

**1.** Réactif immunochimique caractérisé par le fait qu'il comprend une association de deux ou plusieurs séquences polypeptidiques synthétiques choisies parmi :
- une séquence polypeptidique contenant au moins un déterminant antigénique de l'antigène de *gag* de l'isolat λHAT-3 de HTLV-III ;
- une séquence polypeptidique contenant au moins un déterminant antigénique de la glycoprotéine gp120 de l'isolat λHAT-3 de HTLV-III ; et
- une séquence polypeptidique contenant au moins un déterminant antigénique de la glycoprotéine gp41 de l'isolat λHAT-3 de HTLV-III ;
ledit isolat comprenant un ADN génomique provenant du virus HAT-3, isolat de HTLV-III, déposé à ATCC sous le N° 40213.

**2.** Réactif immunochimique selon la revendication 1, caractérisé par le fait qu'il comprend un polypeptide correspondant au polypeptide carboxyterminal long d'environ 58 acides aminés de la protéine gp120.

**3.** Réactif immunochimique selon la revendication 2, caractérisé par le fait qu'il comprend un polypeptide ayant des propriétés immunochimiques comparables à celles d'un polypeptide formé des séquences représentées sur la Figure 9A.

**4.** Réactif immunochimique selon la revendication 1, caractérisé par le fait qu'il comprend un polypeptide correspondant au polypeptide carboxyterminal long d'environ 38 acides aminés de la protéine gp120.

**5.** Réactif immunochimique selon la revendication 4, caractérisé par le fait qu'il comprend un polypeptide ayant des propriétés immunochimiques comparables à celles de l'un quelconque des polypeptides formé des séquences représentées sur la Figure 9B.

6. Réactif immunochimique selon l'une quelconque des revendications 1 à 5, caractérisé par le fait qu'il comprend un polypeptide ayant des propriétés immunochimiques comparables à celles de la séquence d'acides aminés partielle s'étendant entre l'acide aminé N° 53 et l'acide aminé N° 122 de la protéine gp41.

7. Réactif immunochimique selon la revendication 6, caractérisé par le fait qu'il comprend un polypeptide ayant des propriétés immunochimiques comparables à celles d un polypeptide formé des séquences représentées sur la Figure 10, correspondant aux acides aminés N° 571 à 640 de la séquence de HTLV-III.

8. Réactif immunochimique selon l'une quelconque des revendications 1 à 7, caractérisé par le fait qu'il comprend un polypeptide ayant des propriétés immunochimiques comparables à celles de la séquence d'acides aminés partielle s'étendant entre l'acide aminé N° 123 et l'acide aminé N° 172 de la protéine gp41.

9. Réactif immunochimique selon la revendication 8, caractérisé par le fait qu'il comprend un polypeptide ayant des propriétés immunochimiques comparables à celles d'un polypeptide formé des séquences représentées sur la Figure 11A, correspondant aux acides aminés N° 641 à 690 de la séquence de HTLV-III.

10. Réactif immunochimique selon l'une quelconque des revendications 1 à 7, caractérisé par le fait qu'il comprend un polypeptide ayant des propriétés immunochimiques comparables à celles de la séquence d'acides aminés partielle s'étendant entre l'acide aminé N° 123 et l'acide aminé N° 163 de la protéine gp41.

11. Réactif immunochimique selon la revendication 10, caractérisé par le fait qu'il comprend un polypeptide ayant des propriétés immunochimiques comparables à celles d'un polypeptide formé des séquences représentées sur la Figure 11B, correspondant aux acides aminés N° 641 à 681 de la séquence de HTLV-III.

12. Réactif immunochimique selon l'une quelconque des revendications 1 à 11, caractérisé par le fait qu'il comprend un polypeptide ayant des propriétés immunochimiques comparables à celles de la séquence d'acides aminés partielle s'étendant entre l'acide aminé N° 173 et l'acide aminé N° 217 de la protéine gp41.

13. Réactif immunochimique selon la revendication 12, caractérisé par le fait qu'il comprend un polypeptide ayant des propriétés immunochimiques comparables à celles d'un polypeptide formé des séquences représentées sur la Figure 12A, correspondant aux acides aminés N° 691 à 737 de la séquence de HTLV-III.

14. Réactif immunochimique selon l'une quelconque des revendications 1 à 11, caractérisé par le fait qu'il comprend un polypeptide ayant des propriétés immunochimiques comparables à celles de la séquence d'acides aminés partielle s'étendant entre l'acide aminé N° 196 et l'acide aminé N° 217 de la protéine gp41.

15. Réactif immunochimique selon la revendication 14, caractérisé par le fait qu'il comprend un polypeptide ayant des propriétés immunochimiques comparables à celles d'un polypeptide formé des séquences représentées sur la Figure 12B, correspondant aux acides aminés N° 714 à 737 de la séquence de HTLV-III.

16. Réactif immunochimique selon l'une quelconque des revendications 1 à 15, caractérisé par le fait qu'il comprend un polypeptide ayant des propriétés immunochimiques comparables à celles de la séquence d'acides aminés partielle s'étendant entre l'acide aminé N° 30 et l'acide aminé N° 181 de la protéine gp120.

17. Réactif immunochimique selon la revendication 16, caractérisé par le fait qu'il comprend un polypeptide ayant des propriétés immunochimiques comparables à celles d'un polypeptide formé des séquences représentées sur la Figure 8.

EP 0 227 169 B1

**18.** Polypeptide synthétique comprenant au moins une séquence d'acides aminés ayant les propriétés immunochimiques de la séquence d'acides aminés de l'isolat λHAT-3 de HTLV-III, ledit isolat comprenant un ADN génomique provenant du virus HAT-3, isolat de HTLV-III, déposé à ATCC sous le N° 40213, ce polypeptide étant choisi parmi :

- le polypeptide carboxyterminal long de 38 acides aminés de la protéine gp120 ;
- la séquence d'acides aminés partielle s'étendant entre l'acide aminé N° 53 et l'acide aminé N° 122 de la protéine gp41 ;
- la séquence d'acides aminés partielle s'étendant entre l'acide aminé N° 123 et l'acide aminé N° 163 de la protéine gp41 ;
- la séquence d'acides aminés partielle s'étendant entre l'acide aminé N° 196 et l'acide aminé N° 217 de la protéine gp41 ; et
- la séquence d'acides aminés partielle s'étendant entre l'acide aminé N° 30 et l'acide aminé N° 181 de la protéine gp120.

**19.** Polypeptide synthétique selon la revendication 18, comprenant au moins l'une des séquences d'acides aminés représentées sur les Figures 8, 9A, 9B, 10, 11A, 11B, 12A et 12B, correspondant à une partie de la séquence de λHAT-3.

**20.** ADN recombinant caractérisé par le fait qu'il comprend une séquence d'ADN codant pour la production d'un polypeptide selon la revendication 18 ou 19.

**21.** Plasmide caractérisé par le fait qu'il comprend un ADN recombinant selon la revendication 20.

**22.** Cellule-hôte caractérisée par le fait qu'elle est transformée par le plasmide selon la revendication 21.

**23.** Isolat λHAT-3 de HTLV-III, comprenant un ADN génomique provenant du virus HAT-3, isolat de HTLV-III, déposé à ATCC sous le N° 40213.

**24.** Plasmide selon la revendication 21, caractérisé par le fait qu'il est dérivé de l'isolat de HTLV-III de la revendication 23.

**25.** Plasmide de la revendication 24, choisi dans le groupe formé par p24-10SB.3, pENVKPN45, pGAG8, p2-10SB.5, p28-23EB.4 et p28-EB.1.

**26.** Plasmide de la revendication 24, choisi dans le groupe formé par pENVPST1, pENVPSTH3, pENVPSTH3 1-2, pENVPSTH3 2-10, p2-10 EB.3, pENVKPN, pENVKPN24, pENVKPNH3 24, pENVKPNH3 24-10, pENVKPN 28, pENVKPNH3 28, pENVKPNH3 28-23, pENVKPNK3 28-24 et pENVKPNH3 28-45.

**27.** Procédé de préparation du plasmide de la revendication 21, consistant a :
(a) insérer une séquence d'ADNc provenant de HTLV-III dans un plasmide destiné à transformer un hôte cellulaire ;
(b) effectuer dans le plasmide de l'étape (a) la délétion d'une portion d'une séquence identifiable capable d'expression, de façon à produire un décalage de cadre avec déphasage de cette séquence ;
(c) transformer un hôte cellulaire avec le plasmide de l'étape (b) ;
(d) cultiver l'hôte transformé ;
(e) sélectionner les cellules-hôtes présentant des mutations spontanées qui réalisent un décalage de cadre avec mise en phase pour la séquence identifiable de l'étape (b) ;
(f) cultiver la cellule-hôte sélectionnée dans l'étape (e) ;
(g) sélectionner la cellule-hôte qui produit des quantités utiles de polypeptides ayant une immunoréactivité efficace avec des anticorps anti-HTLV-III ; et
(h) isoler et identifier les plasmides qui codent pour les polypeptides de l'étape (g).

**28.** Polypeptide caractérisé par le fait qu'il est exprimé par une cellule-hôte transformée par un plasmide selon la revendication 25 ou 26.

18

29. Nécessaire d'essai à utiliser dans une analyse immunologique, caractérisé par le fait qu'il comprend un réactif immunochimique selon l'une quelconque des revendications 1 à 17.

## HTLY-III (HAT-3)

**FIGURE 1**

Figure 2.1

GAG-1

SEQUENCE OF HAT3 GAG GENE

```
        10          20          30          40          50
        *           *           *           *           *
CCT ATA GTG CAG AAC CTT CAG GGG CAA ATG GTA CAT CAA GCC ATA TCA CCT AGA ACT
Pro Ile Val Gln Asn Leu Gln Gly Gln Met Val His Gln Ala Ile Ser Pro Arg Thr
*                                       *
1                                       10


   60          70          80          90          100         110
   *           *           *           *           *           *
TTA AAT GCA TGG GTA AAA GTA GTA GAA GAG AAG GCT TTT AGC CCA GAA GTA ATA CCC
Leu Asn Ala Trp Val Lys Val Val Glu Glu Lys Ala Phe Ser Pro Glu Val Ile Pro
*                                   *
20                                  30


   120         130         140         150         160         170
   *           *           *           *           *           *
ATG TTT TCA GCA TTA TCA GAA GGA GCC ACC CCA CAA GAT TTA AAC ACC ATG CTA AAC
Met Phe Ser Ala Leu Ser Glu Gly Ala Thr Pro Gln Asp Leu Asn Thr Met Leu Asn
    *                                       *
    40                                      50


       180         190         200         210         220
       *           *           *           *           *
ACA GTG GGG GGA CAT CAA GCA GCC ATG CAA ATG TTA AAA GAG ACT ATC AAT GAG GAA
Thr Val Gly Gly His Gln Ala Ala Met Gln Met Leu Lys Glu Thr Ile Asn Glu Glu
        *                                   *
        60                                  70


230         240         250         260         270         280
*           *           *           *           *           *
GCT GCA GAA TGG GAT AGA GTA CAT CCA GTG CAT GCA GGG CCT ATT GCA CCA CGT CAG
Ala Ala Glu Trp Asp Arg Val His Pro Val His Ala Gly Pro Ile Ala Pro Arg Gln
            *                                   *
            80                                  90


   290         300         310         320         330         340
   *           *           *           *           *           *
ATG AGA GAA CCA AGG GGA AGT GAC ATA GCA GGA ACT ACT AGT ACC CTT CAG GAA CAA
Met Arg Glu Pro Arg Gly Ser Asp Ile Ala Gly Thr Thr Ser Thr Leu Gln Glu Gln
                *                                   *
                100                                 110


   350         360         370         380         390
   *           *           *           *           *
ATA GGA TGG ATG ACA AAT AAT CCA CCT ATC CCA GTA GGA GAA ATC TAT AAA AGG TGG
Ile Gly Trp Met Thr Asn Asn Pro Pro Ile Pro Val Gly Glu Ile Tyr Lys Arg Trp
                    *                                   *
                    120                                 130
```

EP 0 227 169 B1

## Figure 2.2

```
                                                                    GAG-2
400          410          420          430          440          450
*            *            *            *            *            *
ATA ATT CTG GGA TTA AAT AAA ATA GTA AGA ATG TAT AGC CCC ATC AGC ATT CTG GAC
Ile Ile Leu Gly Leu Asn Lys Ile Val Arg Met Tyr Ser Pro Ile Ser Ile Leu Asp
                            *                                    *
                           140                                  150

    460          470          480          490          500          510
    *            *            *            *            *            *
ATA AGA CAA GGA CCT AAG GAA CCC TTT AGA GAC TAT GTA GAC CGG TTC TAT AAA ACT
Ile Arg Gln Gly Pro Lys Glu Pro Phe Arg Asp Tyr Val Asp Arg Phe Tyr Lys Thr
                            *                                    *
                           160                                  170

        520          530          540          550          560          570
        *            *            *            *            *            *
CTA AGA GCC GAG CAA GCT TCA CAG GAT GTA AAA AAT TGG ATG ACA GAA ACC TTG CTG
Leu Arg Ala Glu Gln Ala Ser Gln Asp Val Lys Asn Trp Met Thr Glu Thr Leu Leu
                            *                                    *
                           180                                  190

            580          590          600          610          620
            *            *            *            *            *
GTC CAA AAT GCG AAC CCA GAT TGT AAA ACT ATT TTA AAA GCA TTG GGA CCA GCA GCT
Val Gln Asn Ala Asn Pro Asp Cys Lys Thr Ile Leu Lys Ala Leu Gly Pro Ala Ala
                                    *
                                   200

630          640          650          660          670          680
*            *            *            *            *            *
ACA CTA GAA GAA ATG ATG ACA GCA TGT CAG GGA GTA GGG GGA CCC AGC CAT AAA GCA
Thr Leu Glu Glu Met Met Thr Ala Cys Gln Gly Val Gly Gly Pro Ser His Lys Ala
*                                    *
210                                 220

    690          700          710          720          730          740
    *            *            *            *            *            *
AGA ATT TTG GCT GAA GCA ATG AGC CAA GTA ACA AAT TCA GCT ACC ATA ATG CTG CAG
Arg Ile Leu Ala Glu Ala Met Ser Gln Val Thr Asn Ser Ala Thr Ile Met Leu Gln
    *                                    *
   230                                  240

        750          760          770
        *            *            *
AAA GGT AAT TTT AGG GAC CAA AGA AAA ATT GTT AAG
Lys Gly Asn Phe Arg Asp Gln Arg Lys Ile Val Lys
        *                            *
       260                          269
```

22

## Figure 3.1

ENV-1

SEQUENCE OF HAT3 <u>ENV</u> GENE

```
         10          20          30          40          50
         *           *           *           *           *
ATG AGA GTG ATG GAG ATG AGG AAG AAT TGT CAG CAC TTG TGG AAA TGG GGC ACC ATG
Met Arg Val Met Glu Met Arg Lys Asn Cys Gln His Leu Trp Lys Trp Gly Thr Met
 *                                   *
 1                                   10

     60          70          80          90         100         110
     *           *           *           *           *           *
CTC CTT GGG ATG TTG ATG ATC TGT AGT GCT GCA GAG GAC TTG TGG GTC ACA GTC TAT
Leu Leu Gly Met Leu Met Ile Cys Ser Ala Ala Glu Asp Leu Trp Val Thr Val Tyr
 *                                   *
 20                                  30

        120         130         140         150         160         170
        *           *           *           *           *           *
TAT GGG GTA CCT GTG TGG AAA GAA GCA ACC ACC ACT CTA TTT TGT GCA TCA GAA GCT
Tyr Gly Val Pro Val Trp Lys Glu Ala Thr Thr Thr Leu Phe Cys Ala Ser Glu Ala
     *                                       *
     40                                      50

        180         190         200         210         220
        *           *           *           *           *
AAA GCA TAT AAA ACA GAG GTA CAT AAT GTC TGG GCC AAA CAT GCT TGT GTA CCT ACA
Lys Ala Tyr Lys Thr Glu Val His Asn Val Trp Ala Lys His Ala Cys Val Pro Thr
         *                                   *
         60                                  70

230         240         250         260         270         280
*           *           *           *           *           *
GAC CCC AAC CCA CAA GAA GTA CTA TTG GAA AAT GTG ACA GAA AAT TTT AAC ATG TGG
Asp Pro Asn Pro Gln Glu Val Leu Leu Glu Asn Val Thr Glu Asn Phe Asn Met Trp
             *                                   *
             80                                  90

     290         300         310         320         330         340
     *           *           *           *           *           *
AAA AAT AAC ATG GTA GAA CAG ATG CAT GAG GAT ATA ATC AGT TTA TGG GAT CAA AGC
Lys Asn Asn Met Val Glu Gln Met His Glu Asp Ile Ile Ser Leu Trp Asp Gln Ser
                 *                                       *
                 100                                     110

        350         360         370         380         390
        *           *           *           *           *
CTA AAG CCA TGT GTA AAA TTA ACC CCA CTC TGT GTT ACT TTA AAT TGC ACT GAT GCT
Leu Lys Pro Cys Val Lys Leu Thr Pro Leu Cys Val Thr Leu Asn Cys Thr Asp Ala
                 *                                       *
                 120                                     130
```

EP 0 227 169 B1

Figure 3.2

ENV-2

```
400          410          420          430          440          450
 *            *            *            *            *            *
AAC TTG AAT GGT ACT AAT GTC ACT AGT AGT AGC GGG GGA ACA ATG ATG GAG AAC GGA
Asn Leu Asn Gly Thr Asn Val Thr Ser Ser Ser Gly Gly Thr Met Met Glu Asn Gly
                         *                                        *
                        140                                      150

   460          470          480          490          500          510
    *            *            *            *            *            *
GAA ATA AAA AAC TGC TCT TTC CAG GTC ACC ACA AGT AGA AGA GAT AAG ACG CAG AAA
Glu Ile Lys Asn Cys Ser Phe Gln Val Thr Thr Ser Arg Arg Asp Lys Thr Gln Lys
                             *                                        *
                            160                                      170

      520          530          540          550          560          570
       *            *            *            *            *            *
AAA TAT GCA CTT TTT TAT AAA CTT GAT GTG GTA CCA ATA GAG AAG GGT AAT ATT AGC
Lys Tyr Ala Leu Phe Tyr Lys Leu Asp Val Val Pro Ile Glu Lys Gly Asn Ile Ser
                                 *                                        *
                                180                                      190

         580          590          600          610          620
          *            *            *            *            *
CCT AAG AAT AAT ACT AGC AAT AAT ACT AGC TAT GGT AAC TAT ACA TTG ATA CAT TGT
Pro Lys Asn Asn Thr Ser Asn Asn Thr Ser Tyr Gly Asn Tyr Thr Leu Ile His Cys
                                     *
                                    200

   630          640          650          660          670          680
    *            *            *            *            *            *
AAT TCC TCA GTC ATT ACA CAG GCC TGT CCA AAG GTA TCC TTT GAG CCA ATT CCC ATA
Asn Ser Ser Val Ile Thr Gln Ala Cys Pro Lys Val Ser Phe Glu Pro Ile Pro Ile
 *                                        *
210                                      220

      690          700          710          720          730          740
       *            *            *            *            *            *
CAT TAT TGC ACC CCG GCT GGT TTT GCG ATT CTA AAG TGT AAT GAT AAG AAG TTC AAT
His Tyr Cys Thr Pro Ala Gly Phe Ala Ile Leu Lys Cys Asn Asp Lys Lys Phe Asn
 *                                        *
230                                      240

         750          760          770          780          790
          *            *            *            *            *
GGA ACA GGA CCA TGT AAA AAT GTC AGC ACA GTA CAA TGT ACA CAT GGA ATT AGG CCA
Gly Thr Gly Pro Cys Lys Asn Val Ser Thr Val Gln Cys Thr His Gly Ile Arg Pro
             *                                        *
            250                                      260

800          810          820          830          840          850
 *            *            *            *            *            *
GTA GTG TCA ACT CAA CTG CTG TTA AAT GGC AGT CTA GCA GAA GAA GAG GTA GTA ATT
Val Val Ser Thr Gln Leu Leu Leu Asn Gly Ser Leu Ala Glu Glu Glu Val Val Ile
             *                                        *
            270                                      280
```

24

Figure 3.3

```
                                                                     ENV-3
          860           870           880           890           900           910
           *             *             *             *             *             *
     AGA TCT GAA AAT TTC ACG GAC AAT GTT AAA ACC ATA ATA GTA CAG CTG AAT GCA TCT
     Arg Ser Glu Asn Phe Thr Asp Asn Val Lys Thr Ile Ile Val Gln Leu Asn Ala Ser
                       *                                         *
                      290                                       300
              920           930           940           950           960
               *             *             *             *             *
     GTA CAA ATT AAT TGT ACA AGA CCC AAC AAC AAT ACA AGA AAA AGT ATA ACT AAG GGA
     Val Gln Ile Asn Cys Thr Arg Pro Asn Asn Asn Thr Arg Lys Ser Ile Thr Lys Gly
                           *                                     *
                          310                                   320

      970           980           990          1000          1010          1020
       *             *             *             *             *             *
     CCA GGG AGA GTA ATT TAT GCA ACA GGA CAA ATA ATA GGA GAT ATA AGA AAA GCA CAT
     Pro Gly Arg Val Ile Tyr Ala Thr Gly Gln Ile Ile Gly Asp Ile Arg Lys Ala His
                               *                                 *
                              330                               340

         1030          1040          1050          1060          1070          1080
          *             *             *             *             *             *
     TGT AAC CTT AGT AGA GCA CAA TGG AAT AAC ACT TTA AAA CAG GTA GTT ACA AAA TTA
     Cys Asn Leu Ser Arg Ala Gln Trp Asn Asn Thr Leu Lys Gln Val Val Thr Lys Leu
                                   *                             *
                                  350                           360

            1090          1100          1110          1120          1130          1140
             *             *             *             *             *             *
     AGA GAA CAA TTT GAC AAT AAA ACA ATA GTC TTT ACT TCA TCC TCA GGA GGG GAC CCA
     Arg Glu Gln Phe Asp Asn Lys Thr Ile Val Phe Thr Ser Ser Ser Gly Gly Asp Pro
                                       *                                     *
                                      370                                   380

               1150          1160          1170          1180          1190
                *             *             *             *             *
     GAA ATT GTA CTT CAC AGT TTT AAT TGT GGA GGG GAA TTT TTC TAC TGT AAT ACA ACA
     Glu Ile Val Leu His Ser Phe Asn Cys Gly Gly Glu Phe Phe Tyr Cys Asn Thr Thr
                                       *
                                      390

     1200          1210          1220          1230          1240          1250
      *             *             *             *             *             *
     CAA CTG TTT AAT AGT ACT TGG AAT AGT ACT GAA GGG TCA AAT AAC ACT GGA GGA AAT
     Gln Leu Phe Asn Ser Thr Trp Asn Ser Thr Glu Gly Ser Asn Asn Thr Gly Gly Asn
      *                                         *
     400                                       410

        1260          1270          1280          1290          1300          1310
         *             *             *             *             *             *
     GAC ACA ATC ACA CTC CCA TGC AGA ATA AAA CAA ATT GTA AAC ATG TGG CAG GAA GTA
     Asp Thr Ile Thr Leu Pro Cys Arg Ile Lys Gln Ile Val Asn Met Trp Gln Glu Val
         *                                     *
        420                                   430
```

EP 0 227 169 B1

Figure 3.4

ENV-4

```
      1320          1330          1340          1350          1360
       *             *             *             *             *
GGA AAA GCA ATG TAT GCC CCT CCC ATC AGT GGA CAA ATT AAA TGT ATA TCA AAT ATT
Gly Lys Ala Met Tyr Ala Pro Pro Ile Ser Gly Gln Ile Lys Cys Ile Ser Asn Ile
           *                                    *
          440                                  450

  1370          1380          1390          1400          1410          1420
   *             *             *             *             *             *
ACA GGG CTA CTA TTA ACA AGA GAT GGG GGT GAA GAT ACA ACT AAT ACT ACA GAG ATC
Thr Gly Leu Leu Leu Thr Arg Asp Gly Gly Glu Asp Thr Thr Asn Thr Thr Glu Ile
               *                                        *
              460                                      470

      1430          1440          1450          1460          1470          1480
       *             *             *             *             *             *
TTC AGA CTT GGA GGA GGA AAT ATG AGG GAC AAT TGG AGA AGT GAA TTA TAT AAA TAT
Phe Arg Leu Gly Gly Gly Asn Met Arg Asp Asn Trp Arg Ser Glu Leu Tyr Lys Tyr
                   *                                    *
                  480                                  490

          1490          1500          1510          1520          1530
           *             *             *             *             *
AAA GTG GTA AGA ATT GAA CCA TTA GGA GTA GCA CCC ACT AGG GCA AAG AGA AGA GTG
Lys Val Val Arg Ile Glu Pro Leu Gly Val Ala Pro Thr Arg Ala Lys Arg Arg Val
                       *                                    *
                      500                                  510

  1540          1550          1560          1570          1580          1590
   *             *             *             *             *             *
GTG CAA AGA GAA AAA AGA GCA GTG GGA ACA ATA GGA GCT ATG TTC CTT GGG TTC TTG
Val Gln Arg Glu Lys Arg Ala Val Gly Thr Ile Gly Ala Met Phe Leu Gly Phe Leu
                           *                                    *
                          520                                  530

          1600          1610          1620          1630          1640          1650
           *             *             *             *             *             *
GGA GCA GCA GGA AGC ACT ATG GGC GCA GGC TCA ATA ACG CTG ACG GTA CAG GCC AGA
Gly Ala Ala Gly Ser Thr Met Gly Ala Gly Ser Ile Thr Leu Thr Val Gln Ala Arg
                               *                                    *
                              540                                  550

          1660          1670          1680          1690          1700          1710
           *             *             *             *             *             *
CAC TTA TTG TCT GGT ATA GTG CAA CAG CAA AAC AAT TTG CTG AGG GCT ATT GAG GCG
His Leu Leu Ser Gly Ile Val Gln Gln Gln Asn Asn Leu Leu Arg Ala Ile Glu Ala
                               *                                    *
                              560                                  570

          1720          1730          1740          1750          1760
           *             *             *             *             *
CAA CAG CAT CTG TTG CAA CTC ACG GTC TGG GGC ATC AAA CAG CTC CAG GCA AGA GTC
Gln Gln His Leu Leu Gln Leu Thr Val Trp Gly Ile Lys Gln Leu Gln Ala Arg Val
                               *
                              580
```

26

EP 0 227 169 B1

```
        *                     *                     *                     *                     *
AA AGA TAC CTA AGG GAT CAA CAG CTC CTA GGA ATT TGG GGA TGC TCT
lu Arg Tyr Leu Arg Asp Gln Gln Leu Leu Gly Ile Trp Gly Cys Ser
                                        *
                                       600

          1840          1850          1860          1870          1880
           *             *             *             *             *
TT TGC ACC ACT ACT GTG CCT TGG AAT GCT AGT TGG AGT AAT AAA TCT
le Cys Thr Thr Thr Val Pro Trp Asn Ala Ser Trp Ser Asn Lys Ser
                                        *
                                       620

            1900          1910          1920          1930
             *             *             *             *
TT TGG AAT AAC ATG ACC TGG ATG CAG TGG GAA AGA GAA ATT GAC AAT
le Trp Asn Asn Met Thr Trp Met Gln Trp Glu Arg Glu Ile Asp Asn
                                        *
                                       640

)50          1960          1970          1980          1990
 *             *             *             *             *
`A ATA TAC AAC TTA CTT GAA GAA TCG CAG AAC CAG CAA GAA AAG AAT
 e Ile Tyr Asn Leu Leu Glu Glu Ser Gln Asn Gln Gln Glu Lys Asn
                                        *
;0                                     660

    2010          2020          2030          2040          2050
     *             *             *             *             *
`A TTG GAA TTG GAT AAA TGG GCA AAT TTG TGG AAT TGG TTT GAC ATA
 u Leu Glu Leu Asp Lys Trp Ala Asn Leu Trp Asn Trp Phe Asp Ile
   *                                    *
  670                                  680

          2070          2080          2090          2100
           *             *             *             *
G TGG TAT ATA AGA ATA TTC ATA ATG ATA GTA GGA GGC TTG GTA GGT
u Trp Tyr Ile Arg Ile Phe Ile Met Ile Val Gly Gly Leu Val Gly
  *                                                  *
 690                                                700

)          2130          2140          2150          2160
            *             *             *             *
T TTT GCT GTG CTT TCT ATA GTG AAT AGA GTT AGG CAG GGA TAC TCA
l Phe Ala Val Leu Ser Ile Val Asn Arg Val Arg Gln Gly Tyr Ser
          *                                          *
         710                                        720

  2180          2190          2200          2210          2220
   *             *             *             *             *
` CAG ACC CAC CTC CCA GCC CCG AGG GGA CCC GAC AGG CCC GAA GGA
 e Gln Thr His Leu Pro Ala Pro Arg Gly Pro Asp Arg Pro Glu Gly
             *                                       *
            730                                     740
```

27

```
                                                        ENV-o
     2240          2250          2260          2270       2280
      *             *             *             *          *
GGT GGA GAG AGA GAC AGA GAC AGA TCC GGC GGT GCA GTG AAT GGA
Gly Gly Glu Arg Asp Arg Asp Arg Ser Gly Gly Ala Val Asn Gly
                         *                                 *
                        750                               760


        2300          2310          2320          2330
         *             *             *             *
ATC TGG GAC GAT CTG TGG ACC CTG TGC AGC TTC AGC TAC CAC CGC
Ile Trp Asp Asp Leu Trp Thr Leu Cys Ser Phe Ser Tyr His Arg
                     *
                    770


  ,50          2360          2370          2380          2390
   *             *             *             *             *
CTC TTG ATA GTA GTG AGG ATT GTG GAA CTT CTG GGA CGC AGG GGG
Leu Leu Ile Val Val Arg Ile Val Glu Leu Leu Gly Arg Arg Gly
                             *
                            790


     2410          2420          2430          2440          2450
      *             *             *             *             *
AAG TAT TGG TGG AAT CTC CTG CAG TAT TGG AGT CAG GAG CTA AAG
Lys Tyr Trp Trp Asn Leu Leu Gln Tyr Trp Ser Gln Glu Leu Lys
                             *
                            810


        2470          2480          2490          2500
         *             *             *             *
AGC TTG CTT AAT ACC ACA GCA ATA GCA GTA GCT GAA GGG ACA GAT
Ser Leu Leu Asn Thr Thr Ala Ile Ala Val Ala Glu Gly Thr Asp
                                     *
                                    830


           2530          2540          2550          2560
            *             *             *             *
GTA GCA CAA AGA ATT CTT AGA GCT TTT CTT CAC ATA CCT AGA AGA
Val Ala Gln Arg Ile Leu Arg Ala Phe Leu His Ile Pro Arg Arg
                                         *
                                        850


  2580          2590
   *             *
TA GAA AGG GCT TTG CTG
eu Glu Arg Ala Leu Leu
   *             *
  60             865
```

28

**bla**
**lacI**
**lacPO**

pMLB1113.IG1
Lac Z⁺

EcoRI
SmaI
BamHI
SalI
PstI
HindIII

BamHI
+
DNA
polymeraseI

M13IG

lacZ

ori

**bla**
**lacI**
**lacPO**

pMLB1120.IG1
Lac Z⁻

EcoRI
SmaI
(—)
SalI
PstI
HindIII

M13IG

lacZ

ori

**FIGURE 4**

EP 0 227 169 B1

FIGURE 5

Figure 6

*See Figure 7

EP 0 227 169 B1

EP 0 227 169 B1

Figure 7

32

## Figure 8-1.

```
HAT-3     Ala Glu Asp Leu Trp Val Thr Val Tyr Tyr Gly Val Pro Val
HTLV-III  Thr-----Lys--------------------------------------------------
ARV-2     Thr-----Lys--------------------------------------------------
LAV       Thr-----Lys--------------------------------------------------


Trp Lys Glu Ala Thr Thr Thr Leu Phe Cys Ala Ser Glu Ala Lys Ala
------------------------------------------------------Asp-----Lys----
------------------------------------------------------Asp-----Arg----
------------------------------------------------------Asp-----Lys----


Tyr Lys Thr Glu Val His Asn Val Trp Ala Lys His Ala Cys Val Pro
----Asp---------------------------------Thr---------------------
----Asp---------------------------------Thr---------------------
----Asp---------------------------------Thr---------------------


Thr Asp Pro Asn Pro Gln Glu Val Leu Leu Glu Asn Val Thr Glu Asn
-----------------------------Val-----Val--------------------
-----------------------------Val-----Gly--------------------
-----------------------------Val-----Val--------------------


Phe Asn Met Trp Lys Asn Asn Met Val Glu Gln Met His Glu Asp Ile
-----------------------Asp---------------------His-----------
-----------------------Asn---------------------Gln-----------
-----------------------Asp---------------------His-----------


Ile Ser Leu Trp Asp Gln Ser Leu Lys Pro Cys Val Lys Leu Thr Pro
----------------------------------------------------------------
----------------------------------------------------------------
----------------------------------------------------------------


Leu Cys Val Thr Leu Asn Cys Thr Asp Ala Asn Leu Asn Gly Thr Asn
------------Ser-----Lys------------Leu Lys [ ] Asn Asp--------
------------Thr-----Asn------------Leu [ ] Gly Lys Ala--------
------------Ser-----Lys------------Leu Gly [ ] Asn Ala--------
```

## Figure 8-2

```
Val Thr Ser Ser Ser Gly Gly Thr Met Met Glu Asn Gly [ ] [ ] [ ]
Thr Asn----------Ser Gly Arg Met Ile Met Glu Lys Gly [ ] [ ] [ ]
Thr Asn----------Asn Trp Lys Glu Glu Ile [ ] Lys Gly [ ] [ ] [ ]
Thr Asn----------Asn Thr Asn Ser Ser Ser Gly Glu Met Met Met Glu


[ ] [ ] Glu Ile Lys Asn Cys Ser Phe Gln Val Thr Thr Ser Arg Arg
[ ] [ ]------------------------------Asn Ile Ser----------Ile----
[ ] [ ]------------------------------Asn Ile Thr----------Ile----
Lys Gly----------------------------Asn Ile Ser----------Ile----


Asp Lys Thr Gln Lys Lys Tyr Ala Leu Phe Tyr Lys Leu Asp Val
Gly-----Val----------Glu Tyr-----Phe-----Tyr Lys----------Ile
Asp-----Ile----------Glu Asn-----Leu-----Arg Asn----------Val
Gly-----Val----------Glu Tyr-----Phe-----Tyr Lys----------Ile
```

## Figure 9A

```
HAT-3      Arg Asp Gly Gly Gln Asp Thr Thr Asn Thr Thr Glu Ile Phe
HTLV-III   ----------------Asn Ser Asn [ ]-----Glu Ser-----Ile----
ARV-2      ----------------Thr Asn Val Thr-----Asp Thr-----Val----
LAV        ----------------Asn Asn Asn [ ]-----Glu Ser-----Ile----


Arg Leu Gly Gly Gly Asn Met Arg Asp Asn Trp Arg Ser Glu Leu Tyr
----Pro------------Asp-------------------------------------------
----Pro------------Asp-------------------------------------------
----Pro------------Asp-------------------------------------------


Lys Tyr Lys Val Val Arg Ile Glu Pro Leu Gly Val Ala Pro Thr Arg
-----------------Val Lys--------------------Val-------------Lys
-----------------Ile Lys--------------------Ile-------------Lys
-----------------Val Lys--------------------Val-------------Lys


Ala Lys Arg Arg Val Val Gln Arg Glu Lys Arg Ala
-----------------------------------------------
-----------------------------------------------
-----------------------------------------------
```

## Figure 9B

```
HAT-3      Met Arg Asp Asn Trp Arg Ser Glu Leu Tyr
HTLV-III   ---------------------------------------
ARV-2      ---------------------------------------
LAV        ---------------------------------------


Lys Tyr Lys Val Val Arg Ile Glu Pro Leu Gly Val Ala Pro Thr Arg
-----------------Val Lys--------------------Val-------------Lys
-----------------Ile Lys--------------------Ile-------------Lys
-----------------Val Lys--------------------Val-------------Lys


Ala Lys Arg Arg Val Val Gln Arg Glu Lys Arg Ala
-----------------------------------------------
-----------------------------------------------
-----------------------------------------------
```

Figure 10

```
HAT-3     His Leu Leu Gln Leu Thr Val Trp Gly Ile Lys Gln Leu Gln
HTLV-III  ----------------------------------------------------------
ARV-2     ----------------------------------------------------------
LAV       ----------------------------------------------------------


Ala Arg Val Leu Ala Val Glu Arg Tyr Leu Arg Asp Gln Gln Leu Leu
--------Ile-----------------------------Lys--------------------
--------Val-----------------------------Arg--------------------
--------Ile-----------------------------Lys--------------------


Gly Ile Trp Gly Cys Ser Gly Lys Leu Ile Cys Thr Thr Thr Val Pro
------------------------------------------------Ala--------
------------------------------------------------Ala--------
------------------------------------------------Ala--------


Trp Asn Ala Ser Trp Ser Asn Lys Ser Leu Asn Met Ile Trp Asn Asn
-----------------------------------Glu Gln---------Asn----
-----------------------------------Glu Asp---------Asp----
-----------------------------------Glu Gln---------Asn----


Met Thr Trp Met Gln Trp Glu Arg
----------------Glu-----Asp----
----------------Gln-----Glu----
----------------Glu-----Asp----
```

Figure 11A

```
HAT-3      Glu Ile Asp Asn Tyr Thr Gly Ile Ile Tyr Asn Leu Leu Glu
HTLV-III   --------Asn-------------Ser Leu-----His Ser-----Ile----
ARV-2      --------Asp-------------Asn Thr-----Tyr Thr-----Leu----
LAV        --------Asn-------------Ser Leu-----His Ser-----Ile----


Glu Ser Gln Asn Gln Gln Glu Lys Asn Glu Gln Glu Leu Leu Glu Leu
------------------------------------------------------------------
------------------------------------------------------------------
------------------------------------------------------------------


Asp Lys Trp Ala Asn Leu Trp Asn Trp Phe Asp Ile Thr Gln Trp Leu
----------------Ser--------------------Asn---------Asn--------
----------------Ser--------------------Ser---------Asn--------
----------------Ser--------------------Asn---------Asn--------


Trp Tyr Ile Arg
------------Lys
------------Lys
------------Lys
```

Figure 11B

```
HAT-3      Glu Ile Asp Asn Tyr Thr Gly Ile Ile Tyr Asn Leu Leu Glu
HTLV-III   --------Asn-------------Ser Leu-----His Ser-----Ile----
ARV-2      --------Asp-------------Asn Thr-----Tyr Thr-----Leu----
LAV        --------Asn-------------Ser Leu-----His Ser-----Ile----


Glu Ser Gln Asn Gln Gln Glu Lys Asn Glu Gln Glu Leu Leu Glu Leu
------------------------------------------------------------------
------------------------------------------------------------------
------------------------------------------------------------------


Asp Lys Trp Ala Asn Leu Trp Asn Trp Phe Asp
----------------Ser-------------------Asn
----------------Ser-------------------Ser
----------------Ser-------------------Asn
```

Figure 12A

```
HAT-3     Ile Phe Ile Met Ile Val Gly Gly Leu Val Gly Leu Lys Ile Val
HTLV-III  Leu-------------------------------------------------Arg--------
ARV-2     Ile-------------------------------------------------Arg--------
LAV       Ile-------------------------------------------------Arg--------


Phe Ala Val Leu Ser Ile Val Asn Arg Val Arg Gln Gly Tyr Ser Pro
------------------------------------------------------------------
------------------------------------------------------------------
------------------------------------------------------------------


Leu Ser Phe Gln Thr His Leu Pro Ala Pro Arg Gly Pro Asp Arg Pro
--------------------His---------Ile-------------------------------
--------------------Arg---------Val-------------------------------
--------------------His---------Thr-------------------------------
```

Figure 12B

```
HAT-3     Arg Val Arg Gln Gly Tyr Ser Pro
HTLV-III  -------------------------------
ARV-2     -------------------------------
LAV       -------------------------------


Leu Ser Phe Gln Thr His Leu Pro Ala Pro Arg Gly Pro Asp Arg Pro
--------------------His---------Ile-------------------------------
--------------------Arg---------Val-------------------------------
--------------------His---------Thr-------------------------------
```